# EUROPEAN PATENT SPECIFICATION

(11) **EP 0 714 912 B1**
(45) Date of publication and mention of the grant of the patent: **25.09.2002**
(21) Application number: 95202380.2
(22) Date of filing: 17.07.1991
(51) Int. Cl.: C07K 7/08, A61K 31/70, A61K 39/395, A61K 49/00

(54) **GMP-140 ligand**
GMP-140 Ligand
ligand de GMP-140

(30) Priority: 17.07.1990 US 554199; 05.02.1991 US 650484
(43) Date of publication of application: 05.06.1996
(62) Divisional of application: 91916882.3
(73) Proprietor: THE BOARD OF REGENTS OF THE UNIVERSITY OF OKLAHOMA, Norman, Oklahoma 73019 (US)
(72) Inventor: McEver, Rodger P., Oklahoma City, Oklahoma 73120 (US)
(74) Representative: Hallybone, Huw George

(56) References cited:
- EP-A- 0 344 955
- WO-A-91/06632
- US-A- 4 752 569
- US-A- 4 783 330
- US-A- 4 904 596
- CELL, vol. 56, 1989, XP002000150 G.I. JOHNSTON ET AL.: "Cloning of GMP-140, a Granule Membrane Protein of Platelets and Endothelium: Sequence Similarity to Proteins Involved in Cell Adhesion and Inflammation"
- BLOOD CELLS, vol. 16, 1990, XP002000151 RODGER P. MCEVER: "Properties of GMP-140, an Inducible Granule Membrane Protein of Platelets and Endothelium"
- JIAN-GUO GENG ET AL.: 'Rapid neutrophil adhesion to activated endothelium mediated by GMP-140' NATURE vol. 343, 22 February 1990, XP002000152
- SCIENCE, vol. 249, 1990, XP002000153 JENNIFER R. GAMBLE ET AL.: "Prevention of Activated Neutrophil Adhesion to Endothelium by Soluble Adhesion Protein GMP140"
- CELL, vol. 59, 1989, XP002000154 ERIC LARSEN ET AL.: "PADGEM Protein: A Receptor That Mediates the Interaction of Activated Platelets with Neutrophils and Monocytes"
- THE JOURNAL OF BIOLOGICAL CHEMISTRY, vol. 265, no. 14, 1990, XP002000155 DAVID C. ORD ET AL.: "Structure of the Gene Encoding the Human Leukocyte Adhesion Molecule-1(TQ1, Leu-8) of Lymphocytes and Neutrophils"
- CELL, vol. 63, 1990, XP002000156 ERIC LARSEN ET AL.: "PADGEM-Dependent Adhesion of Platelets to Monocytes and Neutrophils Is Mediated by a Lineage-Specific Carbohydrate, LNF III (CD15)"
- CELL, vol. 63, 1990, XP002000157 B.K. BRANDLEY: "Carbohydrate Ligands of the LEC Cell Adhesion Molecules"
- BIOCHEMICAL AND BIOPHYSICAL RESEARCH, vol. 172, no. 3, 1990, XP002000158 L. CORRAL ET AL.: "Requirement for sialic acid on neutrophils in a GMP-140(PADGEM) mediated adhesive interaction with activated platelets"
- AM. J. RESPIR.CELL MOL.BIOL., vol. 3, 1990, XP002000159 S.D. ROSEN: "The LEC-CAMs: An Emerging Family of Cell Adhesion Receptors Based upon Carbohydrate Recognition"
- BIOCHEMICAL AND BIOPHYSICAL RESEARCH COMM., vol. 164, no. 3, 1989, XP002000160 M.P. SKINNER ET AL.: "Characterization of human platelet GMP-140 as a heparin-binding protein"
- THE JOURNAL OF BIOLOGICAL CHEMISTRY, vol. 259, no. 16, 25 August 1984, pages 10511-10517, XP002017780 YASUO FUKUSHI ET AL: "Novell Fucolipids Accumulating in Human Adenocarcnnoma"
- J.NATIONAL CANCER INST. (JNCI), vol. 78, no. 3, March 1987, pages 479-488, XP002017781 MEI YUAN ET AL: "Expression of Lewis-X and Sialylated Lewis-X Antigens in Human Colorectal Polyps"
- CANCER RESEACH, vol. 46, 1986, pages 2619-2626, XP002017784 REIJI KANNAGI ET AL.: "Quantitative and Qualitative Characterization of Human Cancer-associated Serum Glycoprotein Antigens Expressing Fucosyl or Sialyl-Fucosyl Type 2 Chain Polylactosamine"
- NATIONAL LIBRARY OF MEDICINE, FILE MEDLINE, MEDLINE AN: 86304466, XP002017782 CARLSSON SR ET AL.: "Isolation and Characterization of Leukosialin, a major Sialoglycoprotein of Human Leukocytes" & J. BIOL. CHEM, vol. 261, no. 27, September 1986, pages 12779-12786,
- NATIONAL LIBRARY OF MEDICINE, FILE E, MEDLINE AN: 89196745 STEFAN .ET AL.: "Characterization of a 95 KDa Leucocytesialoglyc XP002018483 & FOLIA BIOL. (PRAHA), vol. 34, no. 4, 1988, pages 255-265,
- R.P. MCEVER: 'A monoclonal antibody to a membrane glycoprotein binds only to activated platelets' THE JOURNAL OF BIOLOGICAL CHEMISTRY vol. 259, no. 15, 10 August 1990, pages 9799 - 9804

## Description

### Background of the Invention

This invention is generally in the field of methods for the treatment and prevention of inflammatory responses involving binding reactions with the selectins, including GMP-140, ELAM-1, and lymphocyte-homing receptor.

The adherence of platelets and leukocytes to vascular surfaces is a critical component of the inflammatory response, and is part of a complex series of reactions involving the simultaneous and interrelated activation of the complement, coagulation, and immune systems.

Endothelium exposed to "rapid" activators such as thrombin and histamine becomes adhesive for neutrophils within two to ten minutes, while endothelium exposed to cytokines such as tumor necrosis factor and interleukin-1 becomes adhesive after one to six hours. The rapid endothelial-dependent leukocyte adhesion has been associated with expression of the lipid mediator platelet activating factor (PAF) on the cell surface, and presumably, the appearance of other endothelial and leukocyte surface receptors. The slower cytokine-inducible endothelial adhesion for leukocytes is mediated, at least in part, by an endothelial cell receptor, ELAM-1, that is synthesized by endothelial cells after exposure to cytokines and then transported to the cell surface, where it binds neutrophils. The isolation, characterization and cloning of ELAM-1 is reviewed by Bevilacqua, et al., in Science 243, 1160-1165 (1989). A peripheral lymph node homing receptor, also called "the murine Mel 14 antigen", "Leu 8", the "Leu 8 antigen" and "LAM-1", is another structure on neutrophils, monocytes, and lymphocytes that binds lymphocytes to high endothelial venules in peripheral lymph nodes. The characterization and cloning of this protein is reviewed by Lasky, et al., Cell 56, 1045-1055 (1989) (mouse) and Tedder, et al., J. Exp. Med. 170, 123-133 (1989).

GMP-140 (granule membrane protein 140), also known as PADGEM, is a cysteine-rich and heavily glycosylated integral membrane glycoprotein with an apparent molecular weight of 140,000 as assessed by sodium dodecyl sulfate polyacrylamide gel electrophoresis (SDS-PAGE). GMP-140 was first purified from human platelets by McEver and Martin, J. Biol. Chem. 259:9799-9804 (1984). The protein is present in alpha granules of resting platelets but is rapidly redistributed to the plasma membrane following platelet activation, as reported by Stenberg, et al., (1985). The presence of GMP-140 in endothelial cells and its biosynthesis by these cells was reported by McEver, et al., Blood 70(5) Suppl. 1:355a, Abstract No. 1274 (1987). In endothelial cells, GMP-140 is found in storage granules known as the Weibel-Palade bodies. GMP-140 (called PADGEM) has also been reported to mediate the interaction of activated platelets with neutrophils and monocytes by Larsen, et al., in Cell 59, 305-312 (October 1989) and Hamburger and McEver, Blood 75:550-554 (1990).

The cDNA-derived amino acid sequence, reported by Johnston, et al., in Cell 56, 1033-1044 (March 24 1989) indicates that it contains a number of modular domains that are likely to fold independently. Beginning at the N-terminus, these include a "lectin" domain, an "EGF" domain, nine tandem consensus repeats similar to those in complement binding proteins, a transmembrane domain (except in a soluble form that appears to result from differential splicing), and a cytoplasmic tail.

When platelets or endothelial cells are activated by mediators such as thrombin, the membranes of the storage granules fuse with the plasma membrane, the soluble contents of the granules are released to the external environment, and membrane bound GMP-140 is presented within seconds on the cell surface. The rapid redistribution of GMP-140 to the surface of platelets and endothelial cells as a result of activation suggests that this glycoprotein could play an important role at sites of inflammation or vascular disruption.

ELAM-1, the homing receptor, and GMP-140 have been termed "selectins", based on their related structure and function.

The *in vivo* significance of platelet-leukocyte interactions has not been studied carefully. However, in response to vascular injury, platelets are known to adhere to subendothelial surfaces, become activated, and support coagulation. Platelets and other cells may also play an important role in the recruitment of leukocytes into the wound in order to contain microbial invasion. Conversely, leukocytes may recruit platelets into tissues at sites of inflammation, as reported by Issekutz, et al., Lab. Invest. 49:716 (1983).

The coagulation and inflammatory pathways are regulated in a coordinate fashion in response to tissue damage. For example, in addition to becoming adhesive for leukocytes, activated endothelial cells express tissue factor on the cell surface and decrease their surface expression of thrombomodulin, leading to a net facilitation of coagulation reactions on the cell surface. In some cases, a single receptor can be involved in both inflammatory and coagulation processes.

Proteins involved in the hemostatic and inflammatory pathways are of interest for diagnostic purposes and treatment of human disorders. However, there are many problems using proteins therapeutically. Proteins are usually expensive to produce in quantities sufficient for administration to a patient. Moreover, there can be a reaction against the protein after it has been administered more than once to the patient. It is therefore desirable to develop peptides having the same, or better, activity as the protein, which are inexpensive to synthesize, reproducible and relatively innocuous. It is also desirable to develop carbohydrate molecules which can be used both *in vitro* and *in vivo* to modulate binding by the selectins, as effectively as the protein molecules, but which are less expensive to synthesize, more reproducible and presumably potentially less likely to cause a reaction.

It is therefore an object of the present invention to provide carbohydrate-based reagents, based on the structure of the leukocyte ligand for GMP-140, to inhibit GMP-140 - mediated adhesive interactions.

It is therefore an object of the present invention to provide the carbohydrate structures forming a part of the receptor for the selectin GMP-140, which is distinct from the other selectins such as ELAM-1.

It is another object of the present invention to provide methods for using these carbohydrate structures to inhibit leukocyte adhesion to endothelium or to platelets.

It is a further object of the present invention to provide methods for using these peptides and carbohydrate structures to modulate the immune response and the hemostatic pathway.

It is yet another object of the present invention to provide carbohydrates and peptides for use in diagnostic assays relating to GMP-140, ELAM-1, and lymphocyte homing receptor.

Peptides derived from three regions of the lectin binding region of GMP-140 have been found to selectively interact with "selectins", including GMP-140, ELAM-1, and lymphocyte homing receptor. The three regions include amino acids 19-34, 54-72, and 66-89, based on the numbers of the residues contained in the peptide, with residue 1 defined as the N terminus of the mature protein after cleavage of the signal peptide. The peptides can be as short as five to eight amino acids in length and are easily prepared by standard techniques.

Fucosylated sialyated lactosamine structures that bind to GMP-140 have also been discovered. The structure is created by expression of α(1,3) fucosyltransferases capable of modifying acceptors containing α(2,3)sialic acid-substituted lactosaminoglycans. Le^{x}, Galβ1,4(Fucαl,3)GlcNAcβ1-R (where R is a protein or other carbohydrate structure), a common trisaccharide structure on myeloid cells but not on lymphocytes or erythroid cells, forms the core of this sialyated structure. The actual structure may be sialyl Le^{x}, which is NeuAcα2-3Galβl-4(Fucα1,3)GlcNAc-R. Other possible structures include difucosyl sialyl Le^{x}, a longer polyfucosylated polyactosaminoglycan, or a related variant. Several of these structures may bind to GMP-140 with various degrees of affinity.

### Summary of the invention.

The present invention provides a glycoprotein comprising a carbohydrate portion comprising a sialylated, fucosylated lactosaminoglycan, the carbohydrate portion O-linked to the glycoprotein, and having affinity binding specific to GMP-140 wherein binding of the glycoprotein ligand to GMP-140 is blocked by the G1 monoclonal antibody to GMP-140 and not blocked by the S12 monoclonal antibody to GMP-140. Monoclonal antibody G1 is clearly disclosed in McEver et al., (J. Biol. Chem., 259, 9799-9804, 1984) and monoclonal antibody S12 is clearly disclosed in Geng et al., (Nature, 343, 757-760, 1999).

Examples demonstrate that the peptides bind neutrophils and inhibit binding of GMP-140 to neutrophils, with an IC₅₀ (the dose required to inhibit adhesion of neutrophils to immobilized GMP-140 by 50%) ranging from 50 to 300 micromolar. The binding affinity can be modulated using sequences from the EGF domain and divalent cations which bind to both the EFG and lectin domains. An assay is also demonstrated that is useful for screening for variations of these peptides that interfere with binding of all selectins or individual selectins, especially GMP-140.

Examples using Chinese Hamster Ovary (CHO) cell lines transfected with specific glycosyl transferases confirm that the oligosaccharide ligand for GMP-140 is a sialylated fucosylated structure, that the sialic acid linkage must be α2, 3 to Gal and that the fucose linkage must be α1, 3 to a GlcNac to which a Gal has been attached by a β1, 4 linkage.

The peptides and carbohydrate structures, including sialyl Le^{x}, difucosyl sialyl Le^{x}, or a longer polyfucosylated polylactosaminoglycan variant, produced synthetically or expressed in genetically engineered cells, are useful as diagnostics and, in combination with a suitable pharmaceutical carrier, for clinical applications in the modulation or inhibition of coagulation processes or inflammatory processes. The peptides and carbohydrate can also be modified to increase *in vivo* half-life, by chemical modification of the amino acids or by attachment to a carrier molecule or inert substrate. Antibodies to these structures can also be used as diagnostics and as pharmaceuticals for modulation of the coagulation or inflammatory processes.

### Brief Description of the Drawings

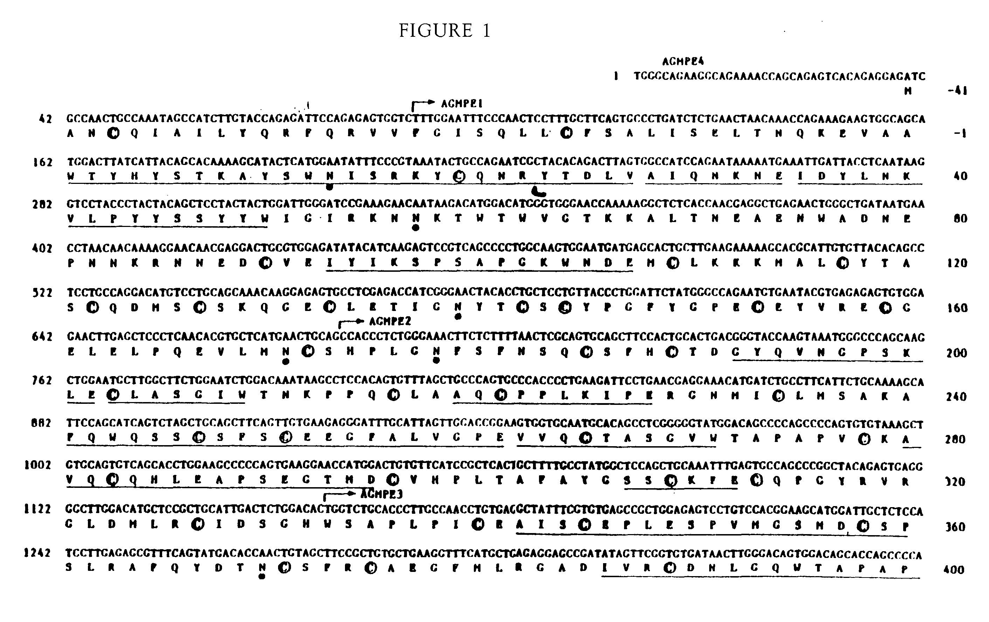

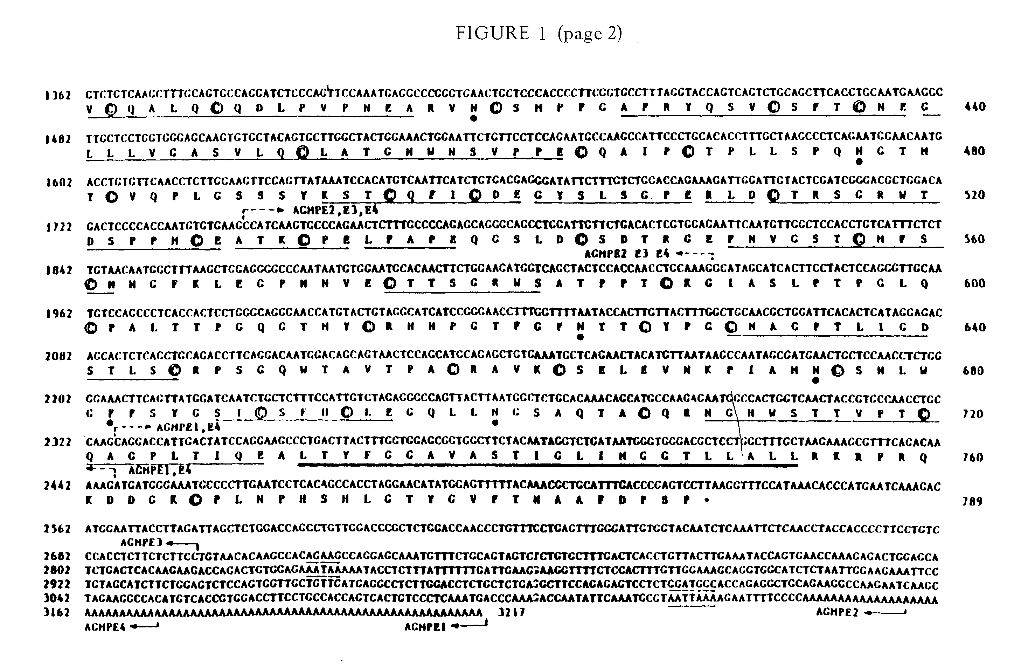

Figure 1 is the nucleotide and deduced amino acid sequence of endothelial cell GMP-140. The translated amino acid sequence of the open reading frame is given in the single-letter code. The stop codon is shown by the asterisk. The thin underlines show the matching positions of amino acid sequences determined from the N-terminus and from 26 peptides of platelet GMP-140. The signal peptide corresponds to positions -41 to -1. The putative transmembrane domain is heavily underlined. The cysteine residues are circled and potential asparagine-linked glycosylation sites (NXS/T) are shown by the dark circles. Two potential polyadenylation signals in the 3' untranslated region are underlined and overlined.

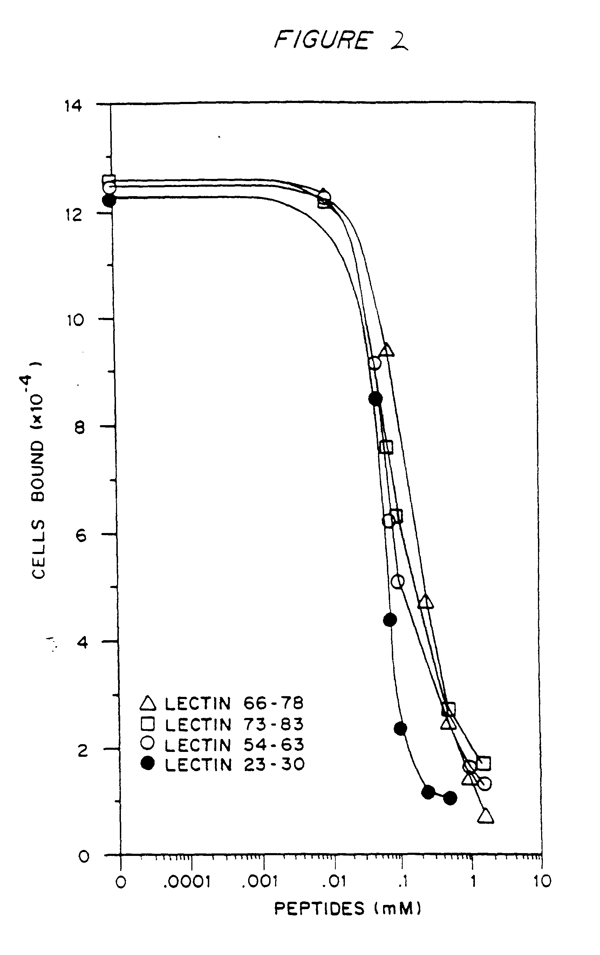

Figure 2 is a graph demonstrating inhibition of binding by peptides from the GMP-140 lectin domain, amino acids 66-78 (triangle), amino acids 73-83 (square), amino acids 54-63 (circle), and amino acids 23-30 (dark circle), comparing number of cells bound to mM of peptides.

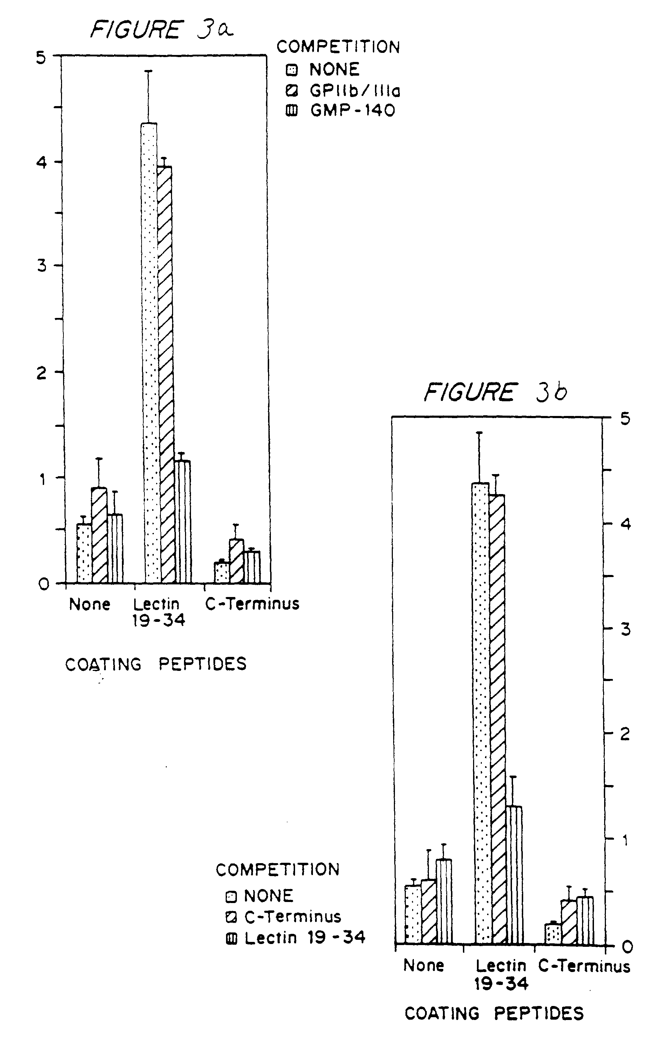

Figure 3A and B are comparisons of the specific adhesion of neutrophils to microtiter wells coated with no peptide (1); coated with lectin domain peptide 19-34 conjugated to KHL (2); or coated with a control carboxyterminus peptide (amino acid residues 761-777) conjugated to KLH (3), blocked with Hank's Balanced Salt Solution containing human serum albumin prior to addition of 2 x 10⁵ neutrophils to each well, in the presence of fluid-phase competitors. Fluid-phase competitors added to the neutrophils prior to transfer to the wells were: Panel A, none (dark bar), purified platelet glycoprotein IIb-IIIa (slashed bar), or purified GMP-140 (stippled bar); Panel B, none (dark bar), 1.5 mM C-terminal peptide 761-777 (slashed bar), 1.5 mM lectin domain peptide 19-34 (stippled bar).

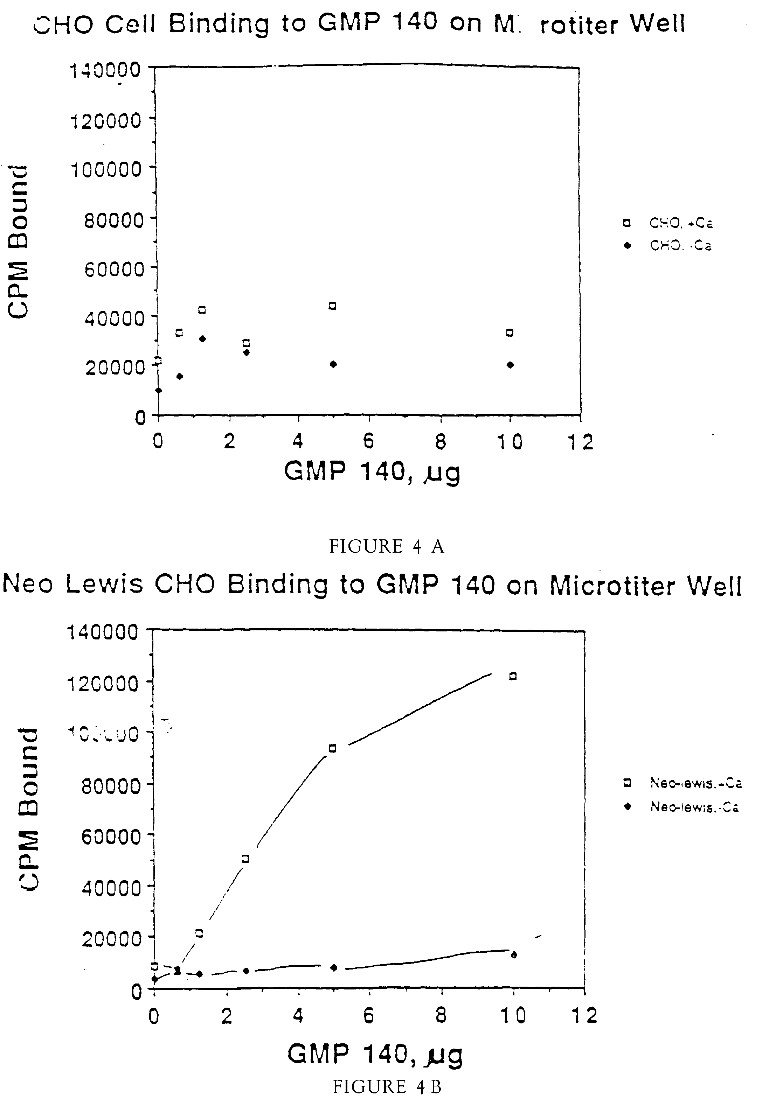

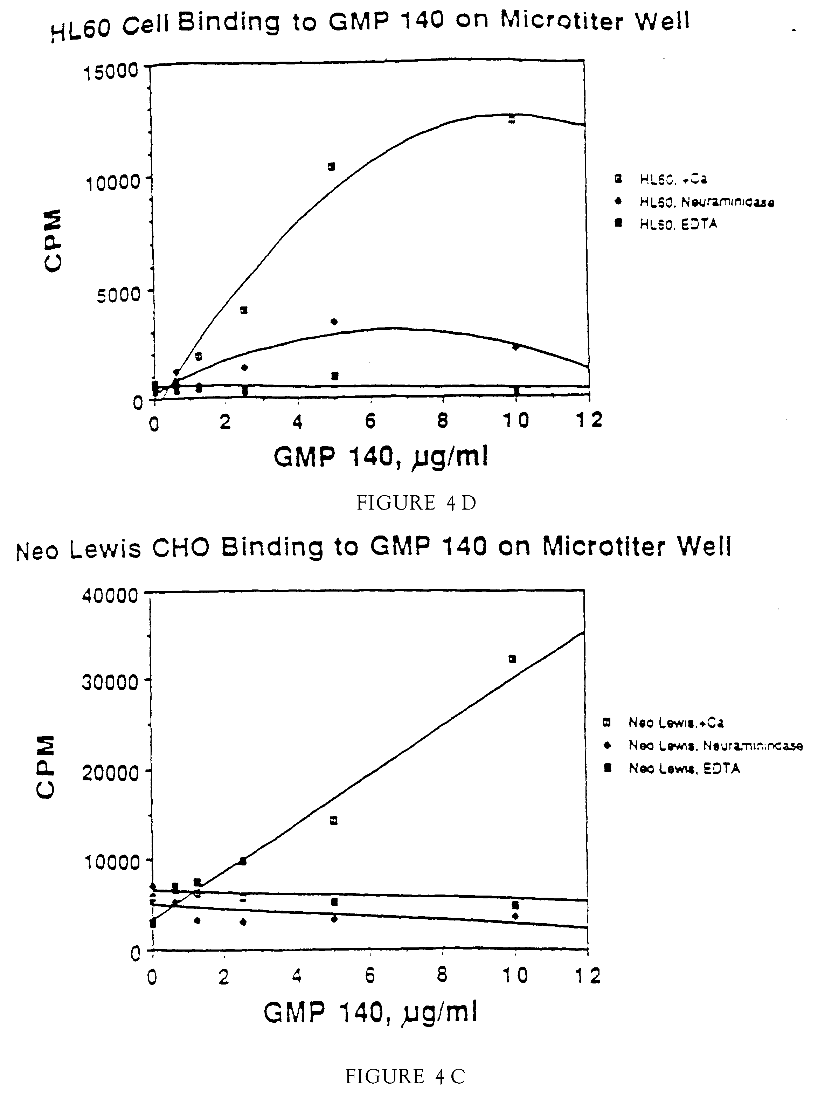

Figures 4A-4D are graphs of the binding of GMP-140 to wild-type and transfected CHO and HL60 cells, measured as CPM ⁵¹Cr, as a function of GMP-140 concentration used to coated the wells (µg GMP-140/ml) , the presence or absence of Ca²⁺, and neuraminidase treatment: Figure 4A, CHO binding to GMP-140, CHO + Ca²⁺ (--squares--), CHO - Ca²⁺ (--dark diamonds--); 4B, Lec 8 CHO binding to GMP-140, Lec 8 + Ca²⁺ (--squares--), Lec 8 - Ca²⁺ (--dark diamonds--); 4C, Neo Lewis CHO binding to GMP-140, Neo Lewis + Ca²⁺ (--squares--), Neo Lewis, Neuraminidase (--dark diamonds--), and Neo Lewis, EDTA (--dark squares--); and 4D, HL60 cell binding to GMP-140, HL60 + Ca²⁺ (--squares--), HL60, Neuraminidase (--dark diamonds--), and HL60, EDTA (--dark squares--).

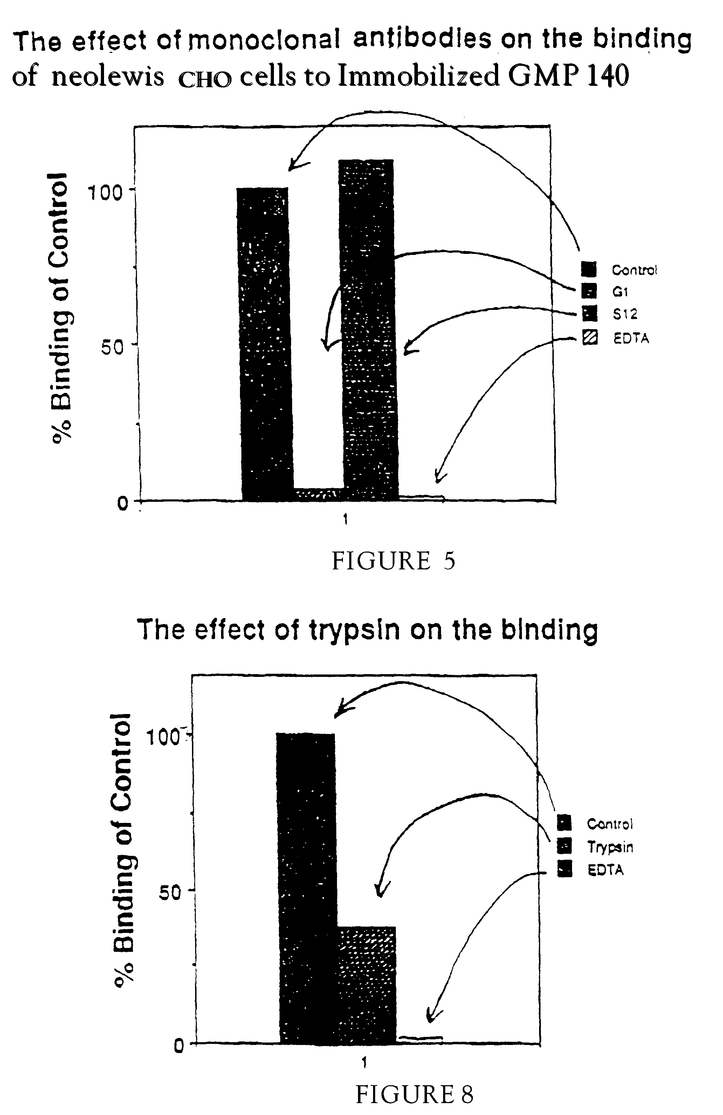

Figure 5 is a graph of the effect of monoclonal antibodies on the binding of NeoLewis CHO cells to immobilized GMP-140, % binding of control for control (dark bar), in the presence of G1 antibody (///), in the presence of S12 antibody (++++), and in the presence of EDTA (/ / /).

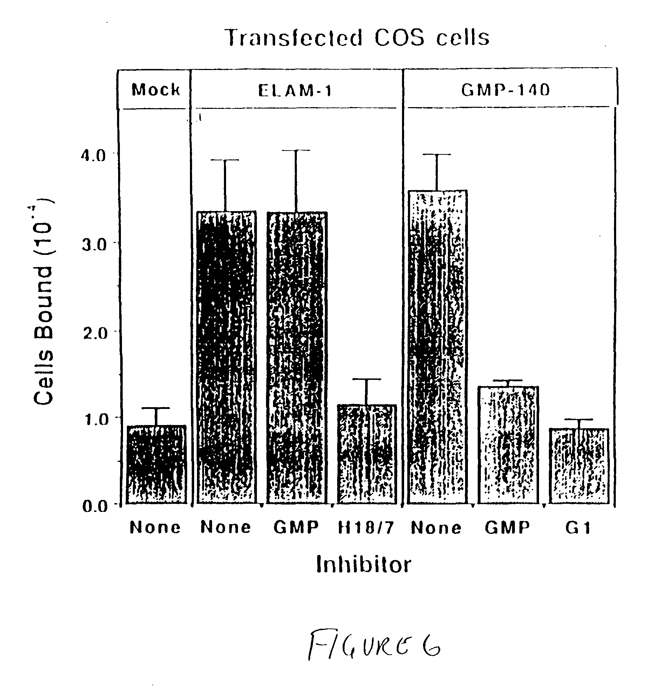

Figure 6 is a graph of transfected COS cells cells bound (10⁻⁴) by soluble ELAM-1 or GMP-140 in a control and in the presence of inhibitors: by ELAM-1 - none, GMP-140, and H18/7 (blocks ELAM-1 binding but not GMP-140 binding); and by GMP-140- none, GMP, and G1.

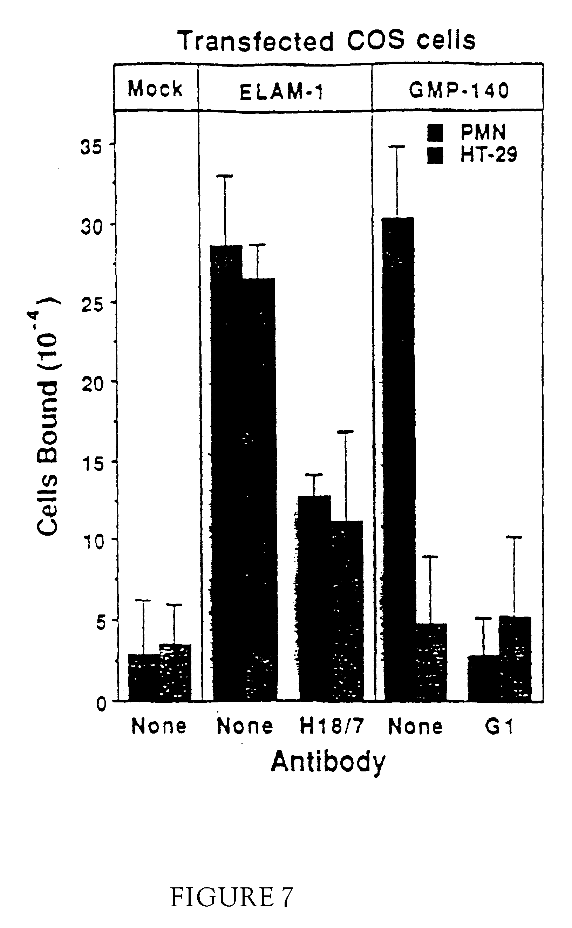

Figure 7 is a graph of the PMS and HT-29 (which express sialyl Le^{x}) cells bound (x 10⁻⁴) by transfected COS cells: control, by ELAM-1 alone and in the presence of H18/7 antibody which blocks binding by ELAM-1 but not GMP-140, and by GMP-140 alone and in the presence of G1 antibody which blocks binding by GMP-140 but not ELAM-1.

Figure 8 is a graph of the effect of trypsin on binding Neo Lewis CHO cells to immobilized GMP-140, % binding of control for control (dark bar), treated with trypsin (///), and in the presence of EDTA (light bar).

### Detailed Description of the Invention

The invention is defined by the technical features of the independent claims appended hereto and follows from the discoveries described below.

The structure and biosynthesis of GMP-140 has now been analyzed in detail. The entire amino acid sequence of GMP-140 has been determined by a combination of protein sequencing of peptide fragments derived from purified platelet GMP-140 and by cloning of cDNAs encoding GMP-140 from a human endothelial cell cDNA library. Based on the structure and on functional studies, GMP-140 acts as a receptor for neutrophils, and interacts with complement protein C3b and the anticoagulant cofactor protein S.

**The cDNA and Amino Acid Sequence of GMP-140.**

Cloning of the gene for GMP-140 was first reported by G.I. Johnston, R.G. Cook and R.P. McEver in Abstract 1238 Supplement II Circulation 78(4) (October 1988). Oligonucleotides were prepared based on N-terminal amino acid sequencing of GMP-140 peptides and used to screen a human endothelial cell cDNA library. A 3.0 kb clone was isolated which encoded a protein of 727 amino acids. An N-terminal domain of 118 residues containing many cysteines, lysines, and tyrosines, which is similar to the asialo glycoprotein receptor, is followed by an EGF-type repeating domain structure, and eight tandem repeats of 62 amino acids each, except for the sixth tandem repeat which has 70 amino acids. The repeats are homologous to those found in a family of proteins that include proteins regulating C3b and C4b, but are unique in having six conserved cysteines per repeat instead of the typical four. These are followed by a 24 amino acid transmembrane region and a 35 amino acid cytoplasmic tail. There appear to be at least two forms of the protein derived by alternative splicing of transcripts of the same gene: a soluble form and a membrane or granule bound form.

The predicted amino acid sequence of endothelial cell GMP-140, shown in Figure 1, suggests the presence of six different structural domains. One of these regions, a 24-residue hydrophobic segment near the C-terminus, is characteristic of membrane spanning domains. Most of the protein is located on the N-terminal side of this domain and appears to be extracytoplasmic, i.e., facing the lumen of the secretory granule, or, following activation of the cell, exposed to the extracellular environment.

Beginning at the N-terminus, the first domain contains 41 residues (labeled -41 to -1 in Figure 1) and has the characteristic features of a signal peptide. These include several positively charged amino acids, followed by a hydrophobic domain and then a region rich in polar residues. The small uncharged residues found at positions -3 and -1 are typical of those found at sites of cleavage by signal peptidase. In addition, the amino acid sequence of the N-terminus of platelet GMP-140 matches the deduced endothelial cell sequence at 25 out of 27 positions from residues 1 to 27.

Following the signal peptide, the translated cDNA sequence predicts a mature protein of 789 residues. Comparison of the sequences of platelet GMP-140 peptides to the deduced endothelial cell sequence showed that 337 of 341 assigned amino acids match, suggesting that both cell types synthesize the same protein.

There are 12 potential asparagine-linked glycosylation sites having the consensus sequence NxS/T. All are located on the extracytoplasmic portion of the molecule and all appear to be glycosylated based on the carbohydrate composition of platelet GMP-140. The mature protein contains 65 cysteines accounting for 8% of the total amino acids. Most of these are predicted to be organized into disulfide bridges since only a small amount of carboxymethyl cysteine can be identified in samples of nonreduced GMP-140 treated with iodoacetamide.

The second domain begins at residue 1 and encompasses the first 118 amino acids of the mature protein. This region is rich in lysine (12%), tyrosine (10%), asparagine (13%), and tryptophan (6%) residues. This region of GMP-140 is designated as a "lectin" domain, since many of the proteins containing this motif bind carbohydrate.

The third domain, which begins at residue 119, has a sequence of 40 amino acids that contains six cysteines. Comparison of this region of GMP-140 to sequences in the NBRF database reveals many proteins that contain the same arrangement of cysteines. The first protein described with this motif is the epidermal growth factor (EGF) precursor, which contains ten homologous copies (Gray, et al., Nature 303:236-240 (1983; Scott, et al., Nature 221:236-240 (1983).

The fourth domain, which begins at residue 159, consists of nine tandem consensus repeats, each containing 62 amino acids; in addition, extensions of eight and four residues, respectively, are found at the ends of the seventh and ninth repeats. The boundaries of this domain are set arbitrarily at the first cysteine of the initial repeat and at the last residue before the putative transmembrane domain. A consensus sequence shows that many amino acids occur in at least five out of the nine repeats. All repeats contain six conserved cysteines, three glycines, and one tryptophan, phenylalanine, proline, and leucine. The cysteine residues are arranged in a different motif than the six cysteines found in the "EGF" domain. The repeats are 31% to 56% identical to each other at the amino acid level and 42% to 62% identical at the nucleotide level. No gaps are required to maximize the alignment among the repeats.

The fifth domain, beginning at residue 731, is the 24-residue putative transmembrane domain. Following this is the sixth domain, a presumed cytoplasmic segment of 35 residues that begins with several highly charged residues and ends at the C-terminus of the protein at residue 789. There are possible phosphorylation sites at serine, threonine, and tyrosine residues, as well as a cysteine that might undergo posttranslational modification.

Two different in-frame deletions are identified among the sequences of the four endothelial-cell clones examined in detail. The first deletion is 186 bp. This deletion removes 62 amino acids from the seventh consensus repeat and predicts a protein containing eight instead of nine repeats. The second deletion is 120 bp, which removes 40 amino acids just after the end of the ninth repeat. The region deleted includes the transmembrane domain and the first few residues of the cytoplasmic domain. The remaining 28 residues at the C-terminus are predicted to continue just after the ninth repeat. A hydrophilicity plot (Kyte and Doolittle, J.Mol.Biol. 157:105-132 (1982) predicts that this form of GMP-140 is soluble.

GMP-140 has been demonstrated to be a receptor on platelets and endothelial cells that binds to a surface ligand on neutrophils and monocytes, thereby facilitating the inflammatory process.

The conclusion that GMP-140 serves as receptor for the adherence of leukocytes to activated endothelial cells and platelets was originally based on several observations: the rapid appearance of GMP-140 on the surface of endothelium stimulated with thrombin or histamine which parallels the inducible adherence of neutrophils to endothelium stimulated with these agonists; the interaction of neutrophils or monocytes with platelets only after platelet activation with agonists such as thrombin which cause redistribution of GMP-140 to the cell surface and not with platelet agonists such as ADP; concentration of GMP-140 in postcapillary venules which are the predominant sites for binding of leukocytes to endothelium prior to their migration across the endothelium into the tissues; specific adherence of purified neutrophils to GMP-140 coated on tissue culture microtiter wells; blocking by polyclonal antibodies to GMP-140 of 60-90% of the adherence of neutrophils to cultured human umbilical vein endothelial cells stimulated with histamine; similarity of the cDNA-derived amino acid sequence of GMP-140 to that of ELAM-1, an endothelial cell protein already known to bind neutrophils, and to lymphocyte homing receptor. Subsequent studies have demonstrated that GMP-140 also mediates adhesion of neutrophils to stimulated platelets, but not unstimulated platelets, in the presence of Ca²⁺. The binding of platelets to neutrophils was inhibited by a monoclonal antibody to GMP-140 and by purified GMP-140.

Other studies have demonstrated that GMP-140 binds to C3b, a complement system protein, and protein S, an anticoagulant cofactor protein. GMP-140 shares sequence homology with the plasma protein C4b-binding protein (C4bp), which not only interacts with the plasma protein C4b but also with protein S.

Peptides derived from GMP-140 have now been discovered that are useful in diagnostics and in modulating the hemostatic and inflammatory responses in a patient wherein a therapeutically effective amount of a peptide capable of blocking leukocyte recognition of GMP-140 is administered to the patient.

It has now been discovered that peptide sequences within the lectin domain of GMP-140, having homology with the lectin domains of other proteins, especially ELAM-1 and the homing receptor, selectively inhibit neutrophil adhesion to purified GMP-140, can therefore be used in diagnostic assays of patients and diseases characterized by altered binding by these molecules, and in screening assays for compounds altering this binding, and should be useful clinically to inhibit or modulate interactions of leukocytes with platelets or endothelial cells involving coagulation and/or inflammatory processes.

The cDNA-derived primary structure of GMP-140 provides several insights into functions for GMP-140 in the vascular system. The most remarkable observation is the striking structural similarity of GMP-140 to two other receptors found on vascular cells which have been recently cloned.

The first of these similar receptors is ELAM-1. ELAM-1 is an endothelial cell protein that is not present in unstimulated endothelium. However, when endothelium is exposed to cytokines such as tumor necrosis factor or interleukin-1, the gene for ELAM-1 is transcribed, producing RNA which in turn is translated into protein. The result is that ELAM-1 is expressed on the surface of endothelial cells 1-4 hours after exposure to cytokines, as reported by Bevilacqua et al., Proc.Natl.Acad.Sci.USA 84:9238-9242 (1987) (in contrast to GMP-140, which is stored in granules and presented on the cell surface within seconds after activation). ELAM-1 has been shown to mediate the adherence of neutrophils to cytokine-treated endothelium and thus appears to be important in allowing leukocytes to migrate across cytokine-stimulated endothelium into tissues. The cDNA-derived primary structure of ELAM-1 indicates that it contains a "lectin" domain, an EGF domain, and six (instead of the nine in GMP-140) repeats similar to those of complement-regulatory proteins, a transmembrane domain, and a short cytoplasmic tail. There is extensive sequence homology between GMP-140 and ELAM-1 throughout the both proteins, but the similarity is particularly striking in the lectin and EGF domains.

The second molecule with overall structural similarity to GMP-140 is a homing receptor found on lymphocytes. Homing receptors are lymphocyte surface structures that allow lymphocytes to bind to specialized endothelial cells in lymphatic tissues, termed high endothelial cells or high endothelial venules (reviewed by Yednock and Rose, Advances in Immunology, vol. 44, F.I. Dixon,ed., 313-378 (Academic Press, New York 1989). This binding allows lymphocytes to migrate across the endothelium into the lymphatic tissues where they are exposed to processed antigens. The lymphocytes then re-enter the blood through the lymphatic system. The homing receptor contains a lectin domain, an EGF domain, two complement-binding repeats, a transmembrane domain, and a short cytoplasmic tail. The homing receptor also shares extensive sequence homology with GMP-140, particularly in the lectin and EGF domains.

A comparison of the lectin domains between GMP-140, ELAM-1, and the homing receptor (LEU-8) is shown in Table I. Based on these sequence similarities it should be possible to select those peptides inhibiting binding of neutrophils to GMP-140 which will inhibit binding of ELAM-1, the homing receptor, and other homologous selectins, to components of the inflammatory process, or, conversely, which will inhibit only GMP-140 binding.

A Fucosylated sialyated lactosamine structure containing glycoprotein that bind to GMP-140 has also been discovered. The structure is created by expression of α(1,3) fucosyltransferases capable of modifying a glycoprotein containing α(2,3)sialic acid-substituted lactosaminoglycan. Le^{x}, Galβ1,4(Fucα1,3)GlcNAcβ1-R (where R is a protein or other carbohydrate structure), a common trisaccharide structure on myeloid cells but not on lymphocytes or erythroid cells, forms the core of this sialyated structure. The actual structure may be sialyl Le^{x}, which is NeuAcα2-3Galβ1-4(Fucα1,3)GlcNAc-R. Other possible structures include difucosyl sialyl Le^{x}, a longer polyfucosylated polyactosaminoglycan, or a related variant. Several of these structures may bind to GMP-140 with various degrees of affinity.

The carbohydrate portion of the ligand is thought to be carried on one or more proteins on myeloid or other cells that interact with GMP-140. The myeloid glycoprotein(s) has not been isolated, but preliminary information about some of its features has been obtained.

This structure was deduced based on studies using protease and neuraminidase digestion, as well as transfected COS cells.

As described in example 5, since binding is at least partially reduced by treating neutrophils with neuraminidase from the Newcastle disease virus, which cleaves sialic acid at α2,3 but not α2,6 linkages, as well as the neuraminidase from *Vibrio cholera,* at least some of the critical linkages in the ligand contain α2,3 linkages. Both types of enzymes also cleave α2,8 linkages, but these are not present on myeloid cells.

Other data with transfected cell lines described in detail in example 6 indicate that a sialyated fucosylated lactosamine with α2,3-linked sialic acid is sufficient for recognition. It is believed the carbohydrate is not Le^{x} alone since an antibody to Le^{x} does not block binding of ¹²⁵I[GMP-140] to neutrophils. Studies comparing binding of GMP-140 with two different multivalent neoglycoconjugates, in which either sialyl Le^{x} or Le^{x} was coupled to bovine serum albumin at a molar ratio of about 10:1, in high concentrations (6.5 µM of conjugate, 65 µM of oligosaccharide) also failed to inhibit binding, suggesting that neither Le^{x} nor sialyl Le^{x} *per se* is the ligand or that the affinity of these oligosaccharides for GMP-140 is too low to measure by this assay.

Evidence indicates that, while the carbohydrate portion of the glycoprotein ligand for GMP-140 is similar or the same as the carbohydrate portion of the glycoprotein ligand for ELAM-1, blocking studies indicate that differences exist in the recognition specificities of GMP-140 and ELAM-1. These may be due to slight differences in oligosaccharide structures recognized by each selectin or to different affinities of binding to the same structure.

It is possible that other oligosaccharide structures not described here may also interact with GMP-140. For example, a sialylated fucosylated lactosaminoglycan with an α2,6 sialic-acid linkage might bind; however, this structure has not been described in eukaryotic cells.

It is possible to manipulate binding of the GMP-140 to the ligand *in vivo* using the sialylated fucosylated lactosamine or a carbohydrate or protein molecule to which multiple sialyated fucosylated lactosamines have been attached. Using multiple sialylated fucosylated lactosamines attached to a single molecule may increase the affinity of the GMP-140 for the artificial molecule over the natural ligand.

The following non-limiting examples further describe the materials, methods, and results leading to these conclusions.

### Comparative example 1: Demonstration of Competitive Inhibition of Binding of Neutrophils to immobilized GMP-140 by Peptides from the lectin domain of GMP-140.

The role of the GMP-140 lectin domain was tested by synthesizing a series of peptides spanning almost all of the 118 residues of the lectin domain, except for two hydrophobic stretches predicted to be sequestered in the interior of the molecule. Peptides were also synthesized encompassing the EGF-like domain (36 residues) which follows the lectin domain. Peptides were also made from one of the consensus repeats, the transmembrane region, and the C-terminus (cytoplasmic tail) of the molecule as controls. Active peptides derived from the lectin domain are shown in Table I which also aligns the related sequences of the lectin domains of ELAM-1 and the homing receptor, LEU-8. Peptides were prepared either on an Applied Biosystems Model 430A automated peptide synthesizer using t-Boc chemistry or on a Dupont RAMPS manual peptide synthesizer using Fmoc chemistry. After cleavage from the resin on which they were synthesized, all peptides were purified by reverse phase high performance liquid chromatography.

Peptides were screened for their ability to inhibit neutrophil adhesion to purified GMP-140 immobilized on plastic wells, using the assay described by Geng, et al., Nature 343, 757-760 (1990).

Human neutrophils are isolated from heparinized whole blood by density gradient centrifugation on Mono-Poly resolving media, Flow Laboratories. Neutrophil suspensions are greater than 98% pure and greater than 95% viable by trypan blue exclusion. For adhesion assays, neutrophils are suspended at a concentration of 2 x 10⁶ cells/ml in Hanks' balanced salt solution containing 1.26 mM Ca²⁺ and 0.81 mM Mg²⁺ (HBSS, Gibco) with 5 mg/ml human serum albumin (HBSS/HSA). Adhesion assays are conducted in triplicate in 96-well microtiter plates, Corning, incubated at 4°C overnight with 50 microliters of various protein solutions.

GMP-140 is isolated from human platelet lysates by immunoaffinity chromatography on antibody S12-Sepharose™ and ion-exchange chromatography on a Mono-Q™ column (FLPC, Pharmacia Fine Chemicals), as follows.

Outdated human platelet packs (100 units) obtained from a blood bank and stored at 4°C are pooled, adjusted to 5 mM EDTA at pH 7.5, centrifuged at 4,000 rpm for 30 min in 1 liter bottles, then washed three times with 1 liter of 0.1 M NaCl, 20 mM Tris pH 7.5 (TBS), 5 mM EDTA, 5 mM benzamidine.

The pellets are then resuspended in a minimum amount of wash buffer and made 1 mM in DIFP, then frozen in 50 ml screwtop tubes at -80°C.

The frozen platelets are thawed and resuspended in 50 ml TBS, 5 mM benzamidine, 5 mM EDTA pH 7.5, 100 M leupeptin. The suspension is frozen and thawed two times in a dry ice-acetone bath using a 600 ml lyophilizing flask, then homogenized in a glass/teflon mortar and pestle and made 1 mM in DIFP. The NaCl concentration is adjusted to 0.5 M with a stock solution of 4 M NaCl. After stirring the suspension at 4°C, it is centrifuged in polycarbonate tubes at 33,000 rpm for 60 min at 4°C. The supernatant (0.5 M NaCl wash) is removed and saved; this supernatant contains the soluble form of GMP-140. Care is taken not to remove the top part of the pellet with the supernatant. The pellets are then homogenized in extraction buffer (TBS, 5 mM benzamidine, 5 mM EDTA, pH 7.5, 100 M leupeptin, 2% Triton X-100). After centrifugation at 19,500 rpm for 25 min at 4°C, the supernatant is removed. The extraction procedure is repeated with the pellet and the supernatant is combined with the first supernatant. The combined extracts, which contain the membrane form of GMP-140, are adjusted to 0.5 M NaCl.

The soluble fraction (0.5 M NaCl wash) and the membrane extract (also adjusted to 0.5 M NaCl) are absorbed with separate pools of the monoclonal antibody S12 (directed to human GMP-140) previously coupled to Affigel (Biorad) at 5 mg/ml for 2 h at 4°C. After letting the resins settle, the supernatants are removed. The S12 Affigel containing bound GMP-140 is then loaded into a column and washed overnight at 4°C with 400 ml of 0.5 M NaCl, 20 mM Tris pH 7.5, 0.01% Lubrol PX.

Bound GMP-140 is eluted from the S12 Affigel with 100 ml of 80% ethylene glycol, 1 mM MES pH 6.0, 0.01% Lubrol PX. Peak fractions with absorbance at 280 nm are pooled. Eluates are dialyzed against TBS with 0.05% Lubrol, then applied to a Mono Q column (FPLC from Pharmacia). The concentrated protein is step eluted with 2 M NaCl, 20 mM Tris pH 7.5 (plus 0.05% Lubrol PX for the membrane fraction). Peak fractions are dialyzed into TBS pH 7.5 (plus 0.05% Lubrol PX for the membrane fraction).

GMP-140 is plated at 5 micrograms/ml and the control proteins: human serum albumin (Alb), platelet glycoprotein IIb/IIIa (IIb), von Willebrand factor (vWF), fibrinogen (FIB), thrombomodulin (TM), gelatin (GEL) or human serum (HS), are added at 50 micrograms/ml. All wells are blocked for 2 h at 22°C with 300 microliters HBSS containing 10 mg/ml HSA, then washed three times with HBSS containing 0.1% Tween-20 and once with HBSS. Cells (2 x 10⁵ per well were added to the wells and incubated at 22°C for 20 min. The wells were then filled with HBSS/HSA, sealed with acetate tape (Dynatech), and centrifuged inverted at 150 g for 5 min. After discarding nonadherent cells and supernates, the contents of each well are solubilized with 200 microliters 0.5% hexadecyltrimethylammonium bromide, Sigma, in 50 mM potassium phosphate, pH 6.0, and assayed for myeloperoxidase activity, Ley, et al., Blood 73, 1324-1330 (1989). The number of cells bound was derived from a standard curve of myeloperoxidase activity versus numbers of cells. Under all assay conditions, the cells released less than 5% of total myeloperoxidase and lactate dehydrogenase. Results are presented in Table I. 100% adhesion is that seen in the presence of the C-terminal peptide (amino acid residues 761-777 of GMP-140) as a negative control; this value is the same as controls in which neither peptide nor antibody is added to the cells. Inhibition is read as a lower percent adhesion, so that a value of 5% means that 95% of the specific adhesion was inhibited.

None of the peptides from the EGF domain of GMP-140 inhibited adhesion. However, peptides from three noncontiguous regions of the lectin domain inhibited adhesion. The three regions from the lectin domain are from amino acid 19 to 34, amino acid 54 to 72, amino acid 73 to 89, and an overlapping peptide of amino acids 66-78. Amino acids are numbered based on the number of the residues contained in the peptide, with residue 1 defined as the N terminus of the mature protein after cleavage of the signal peptide.

Currently, the shortest peptide sequences derived from these sequences that are known to have activity range from eight to thirteen amino acids, varying somewhat depending on the area of the lectin domain from it was derived. Some of the shorter peptides have more activity than the longer peptide sequences. The shortest active peptide characterized at this time is lectin domain amino acids 23 to 30, derived from lectin domain amino acids 19 to 34. This peptide is identical among GMP-140, ELAM-1, and the homing receptor with the exception of a single amino acid difference in ELAM-1, and is therefore expected to inhibit cell-cell contacts mediated by all three selectins. The shortest active peptide derived from lectin domain amino acids 54 to 72 known at this time is from amino acid 54 to 63. The shortest active peptide derived from lectin domain amino acids 78-89 known at this time is from amino acids 73 to 83. In addition, an overlapping peptide, spanning amino acids 66 to 78, is very active. It may be possible to design two active shorter, non-overlapping peptides from the region spanning amino acids 66 to 83.

As shown in Table I, some of these regions are more highly conserved among the selectins than others. As a result, it is possible to use peptides derived from the highly conserved regions to modulate interactions involving all of the selectins and peptides from the regions that are less conserved among the selectins to modulate interactions only involving GMP-140. For example, the central core of lectin region 19-34 (residues 23-30) is extraordinarily conserved among the three molecules. In contrast, the amino acid sequence 54 to 60, derived from lectin region 54 to 72, has a number of differences among the selectins.

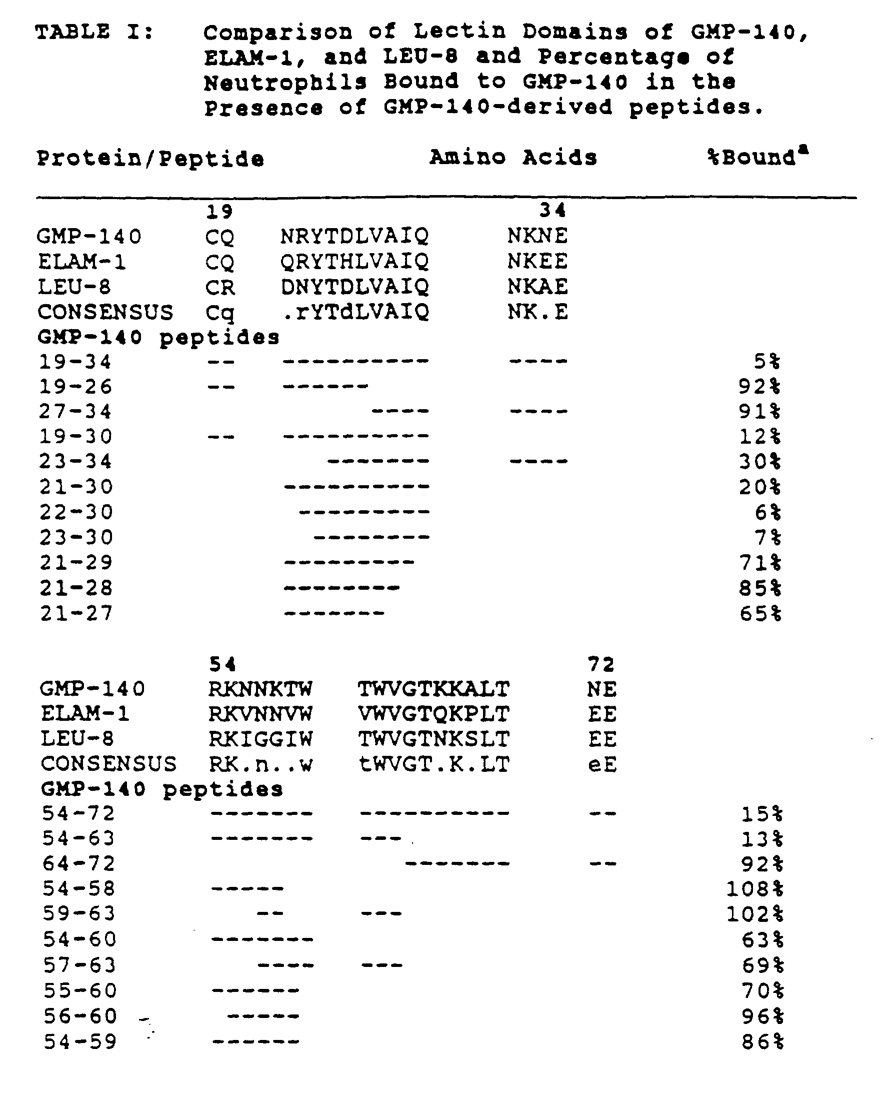

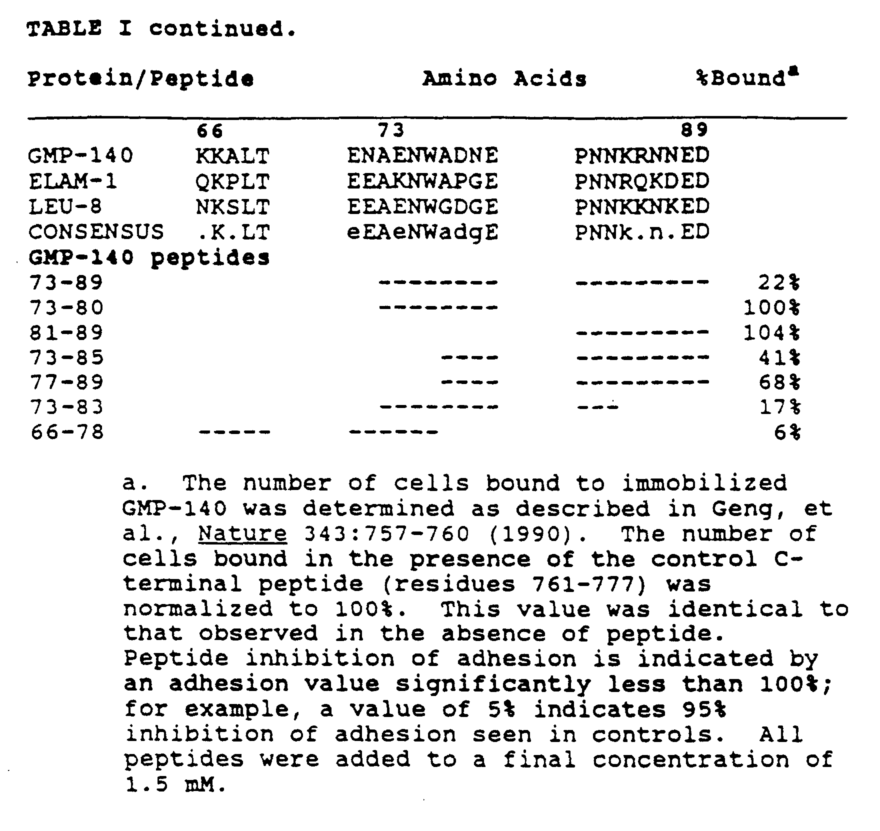

### Comparative example 2: Demonstration of Competitive Inhibition by Peptides from the lectin domain of GMP-140 of the binding of monoclonal antibodies to immobilized GMP-140.

Example 1 demonstrates that peptides from three regions of the lectin domain of GMP-140 inhibit binding of neutrophils to GMP-140 immobilized on a surface. A study was also done to determine whether the peptides would also inhibit binding of monoclonal antibodies to the immobilized GMP-140.

Three monoclonal antibodies (mAb) that block adhesion of neutrophils to GMP-140 were developed, designated G1, G2, and G3. Based on competitive ELISAs with the purified protein, G1, G2, and G3 each recognize distinct or only partially overlapping epitopes. 1.5 mM peptide was added to biotinylated mAb at a concentration of 2.5 micrograms/ml, which were then added to the wells containing GMP-140, as described in example 1. Binding was measured by an ELISA with an avidin detection system.

The monoclonal antibodies were biotinylated as follows: to 0.5 ml of purified IgG antibody (1 mg/ml in PBS, pH 7.4) was added 50 µl of 3.2 mM N-hydroxysuccinimide biotin in dimethyl sulfoxide and 50 µl of 1 M NaHCO₃. After a 2 hour incubation at room temperature in the dark, the reaction was stopped with 50 µl of 1 M NH₄Cl. The biotinylated antibody was then separated from other components by gel filtration on a PD-10 column equilibrated in PBS. The ELISA was performed as follows; all steps were performed at room temperature.

Biotinylated antibody (2.5 µg/ml) with or without 1.5 mM peptide was incubated with wells coated with GMP-140 as described in example 1. After a 2 hour incubation, the antibody was removed, the wells were washed, and 0.1 ml of horseradish peroxidase-conjugated stepavidin (Pierce), diluted 1:1,000 in HBSS/HSA, was added for 30 min. The wells were then washed and 0.1 ml of peroxidase substrate (Pierce) was added for 15 min. The color reaction was read at 405 nm.

The results are shown in Table II.

**TABLE II:**

| **Effect of soluble peptides on binding of biotinylated monoclonal antibodies to immobilized GMP-140.** | | | |
|---|---|---|---|
| **PEPTIDES**^{**a**} | **% Binding of Biotinylated MAbs**^{**b**} | | |
| | **G1** | **G2** | **G3**^{**c**} |
| LECTIN 19-34 | 23 | 26 | 24 |
| LECTIN 19-30 | | | 54 |
| LECTIN 54-72 | 106 | 125 | 104 |
| LECTIN 73-89 | 95 | 99 | 94 |
| LECTIN 91-108 | 97 | 93 | 96 |
| LECTIN 110-116 | 98 | 100 | 108 |
| LECTIN/EGF 118-126 | 92 | 92 | 104 |
| EGF 128-132 | 107 | 92 | 104 |
| EGF 134-141 | 97 | 104 | 109 |
| EGF 145-158 | 94 | 92 | 108 |
| REPEATS 544-556 | 107 | 96 | 111 |
| TRANSMEMBRANE 755-761 | 100 | 119 | 104 |
| SECRETORY 720-721/762-765 | 106 | 99 | 104 |
| C-TERMINUS 761-777 | 100 | 100 | 100 |

| | | | |
|---|---|---|---|
| a. The peptide (1.5 mM) was added to the mAb (2.5 micrograms/ml) prior to addition of the solution to the microtiter well containing immobilized GMP-140. | | | |
| b. Binding of the biotinylated mAb G1, G2, or G3 to immobilized GMP-140 was measured by ELISA with an avidin detection system. | | | |
| c. G1, G2, and G3 are antibodies binding to GMP-140 that prevent neutrophil adhesion to immobilized GMP-140. | | | |

### Comparative example 3: Effect of Concentration on Peptide Inhibition of Binding of Neutrophils to Immobilized GMP-140.

Peptides from the lectin-like domain of GMP-140 were assayed for their ability to inhibit adhesion of neutrophils to immobilized GMP-140 in the assay described in example 1. Concentrations tested ranged from 0.1 mM to 1.5 mM.

The results for four peptides are shown in Figure 2. It is apparent that the peptides from the GMP-140 lectin domain, amino acids 66-78, amino acids 73-83, amino acids 54-63, and amino acids 23-30, inhibited binding in a dose-dependent manner. The IC₅₀, the dose of peptide required to inhibit adhesion by 50%, ranges from about 50 µM to about 300 µM, depending on the peptides. These ranges are well within the effective concentrations for the in *vivo* administration of peptides, based on comparison with the RGD-containing peptides, described, for example, in U.S. Patent No. 4,792,525 to Ruoslaghti, et al., used *in vivo* to alter cell attachment and phagocytosis.

### Comparative example 4: Modification of Peptides and Comparison of Adhesion Potency with GMP-140.

In some cases, modification of the peptides by alteration of the amino acids themselves or by attachment to a carrier molecule is required to increase half-life of the molecule *in vivo.*

Lectin peptide 19-34 was conjugated to the carrier protein keyhole limpet hemocyanin by its N-terminal cysteine by standard procedures such as the commercial Imject kit from Pierce Chemicals. This peptide-KLH conjugate was then tested in the assay described in example 1 and the numbers of cells bound determined.

Figures 3A and B are comparisons of the specific adhesion of neutrophils to microtiter wells coated with no peptide (1); coated with lectin domain peptide 19-34 conjugated to KHL (2); or coated with a control carboxyterminus peptide (amino acid residues 761-777) conjugated to KLH (3), blocked with Hank's Balanced Salt Solution containing human serum albumin prior to addition of 2 x 10⁵ neutrophils to each well, in the presence of fluid-phase competitors. Fluid-phase competitors added to the neutrophils prior to transfer to the wells were none (control), purified platelet glycoprotein IIb-IIIa (control), or purified GMP-140 (Panel A); or none (control), 1.5 mM C-terminal peptide 761-777 (control), 1.5 mM lectin domain peptide 19-34 (Panel B).

It is apparent from the graphs that the lectin peptide-KHL conjugate, when immobilized on plastic, directly supports neutrophil adhesion, although not as efficiently as native GMP-140. For purposes of comparison, when 200,000 neutrophils are added to a well, the lectin peptide 19-34 binds 40,000 and GMP-140 binds 120,000. The peptide is coupled to KLH simply to facilitate its coating to plastic. The ability to compete for adhesion with fluid-phase purified GMP-140 and the lectin peptide 19-34, but not with other proteins or peptides, shows that the adhesion is specific. In other studies, a large number of peptides from other regions of the lectin domain, the EGF domain, and scattered regions of GMP-140 do not inhibit neutrophil adhesion to immobilized GMP-140.

### Example 5: Characterization of the "Ligand" or "Counter-receptor" for GMP-140 by enzyme digestion.

The lectin 19-34 peptide prevents binding to GMP-140 of all three monoclonal antibodies that block interactions of GMP-140 with leukocytes. This provides additional proof of the importance of the lectin domain in leukocyte recognition. It is postulated from this data that the conformation of the lectin domain is modulated by interactions with the EGF domain; these interactions in turn are modulated by divalent cations, which may bind to both the lectin and EGF domains. The result is a three-dimensional conformation of the lectin domain that confers affinity and specificity of binding to its receptor(s) on neutrophils and monocytes.

Neutrophils were isolated, suspended to 4 x 10⁶/ml in Ca²⁺/Mg²⁺-free HBSS, supplemented with 1 mg/ml HSA and 1 mM Ca²⁺ (HBSS/HSA/Ca), and kept at 4°C until used.

Neutrophils were treated with proteases, either trypsin or elastase, to determine if the receptor contains a protease-sensitive protein component. Neutrophils, suspended in HBSS/10 mM MOPS, pH 7.5 (HBSS/MOPS), were treated twice at 22°C for 10 min with 2 mM diisopropylfluorophosphate (DFP) to inactivate endogenous serine proteases. The cells were then washed with HBSS/MOPS and fixed with one-eight volume of 8% paraformaldehyde for 30 min at 22°C, followed by addition of one-eighth volume of 0.5 M glycine/0.25 M Tris, pH 7.5. Fixed neutrophils (7.5 x 10⁶/ml) in HBSS/MOPS, were incubated at 37°C with TPCK-trypsin (0.77 µM, 41 U/ml) for 10 min or with elastase (40 µM, 7.8 U/ml) for 30 min. Control cells were incubated under identical conditions with the same concentrtions of enzyme previously inactivated with DFP or buffer alone. After the incubation period, DFP was added to 2 mM and the cells were pelleted at 400 g for 5 min. The cells were resuspended with HBSS/MOPS and DFP was added again to 2 mM. After centrifugation at 400 g for 5 min, the cell pellets were resuspended to 4 x 10⁶/ml in HBSS/human serum albumin (HSA)/Ca and specific binding of [¹²⁵I]GMP-140 was determined. DFP-treated fixed neturophils (1.6 x 10⁷/ml) in 0.15 M NaCl, 50 mM acetate, pH 6.0, 10 mg/ml HSA, 9 mM CaCl₂, 0.05% sodium azide (digestion buffer) were incubated with neuraminidase, endo-β-galactosidase, or buffer for varying time periods at 37°C in the presence of 20 µM leupeptin, 30 µM antipain, 0.64 mM benzamidine, and 100 KIU/ml aprotinin. In some incubations, 0.5 to 20 mM of the neuraminidase inhibitor Neu2en5Ac, dissolved in digestion buffer, was included. At these concnetrations the pH of the reaction mixture was not affected by the inhibitor. After enzyme treatment, the cells were washed twice with cold HBSS/HSA/Ca and resuspended in HBSS/HSA/Ca to 4 x 10⁶/ml before measurement of [¹²⁵I] GMP-140 binding. The NDV neuraminidase used was a suspension of virus particles, each of which contains approximately 10³ neuraminidase molecules, whereas the V. *cholerae* enzyme is in solution.

Binding of [¹²⁵I]GMP-140 to neutrophils was decreased to 4 to 5% by the proteases, but not by elastase or trypsin inactivated with diisopropylfluorophosphate, indicating that at least a substantial fraction of the leukocyte counter-receptors for GMP-140 contain, or are associated with, a protease-sensitive protein component.

Neutrophils were treated with neuraminidase from either *Vibrio cholera* (Boehringer-Mannheim Biochemicals, Indianapolis, IN) or Newcastle disease virus (NDV) (isolated as described by Paulson, J.C., et al., J. Biol. Chem. 254: 2120-2124 (1979)) and endo-β-galactosidase from *Bacteroides fragilis* (Boehringer-Mannheim). Neuraminidase from V. cholera cleaves α2-3-, α2-6-, and α2-8-linked sialic acids. NDV neuraminidase cleaves only α2-3- and α2-8- linked sialic acids.

Treatment of neutrophils with neuraminidase purified from *Vibrio cholera* greatly decreased both binding of ¹²⁵[GMP-140] to human neutrophils and adhesion of neutrophils to immobilized GMP-140, indicating that sialic acid residues constitute an essential component of the leukocyte counter-receptor(s) for GMP-140. After 10 to 30 min of incubation with 0.1 to 0.2 U/ml of *V. cholera* or NDV neuraminidase, specific GMP-140 binding decreased to 28 ± 9 and 52 ± 9% (mean ± SD, n = 7), respectively, when compared with sham-treated controls. To minimize the possibility that this effect was due to either endogenous neutrophil proteases or protease contamination in the neuraminidase preparations, neutrophils were treated with DFP before fixation to inactivate endogenous serine proteases, and the neuraminidase incubations were performed in the presence of 10 mg/ml HSA as well as several protease inhibitors. The specificity of the neuraminidase effect was further demonstrated by the ability of a competitive neuraminidase inhibitor, Neu2en5Ac, to prevent the neuraminidase-induced reduction in GMP-140 binding to neutrophils. Neu2en5Ac inhibited the effect of neuraminidase in a dose-dependent manner with an IC₅₀ of 2.5 mM.

These results indicate that the counter-receptor, or ligand, on leukocytes for GMP-140 is a glycoprotein wherein sialic acid is required for receptor function. Neutrophils contain both α2-3- and α2-6-linked sialic acids, but α2-8-linkages have not been detected. Partial loss of GMP-140 binding after treatment with NDV neuraminidase implies that at least some of the sialic acid linkages in the receptor are of the α2-3- type. The greater inhibition observed using the V. *cholera* enzyme may mean that α2-6-linkages are also required for receptor function, or the results may be due to lack of accessibility of all of essential linkages by the NDV enzyme, which is part of an intact virus.

Myeloid cells, in contrast to erythroid cells and lymphoid cells, are rich in polylactosaminoglycans which can terminate in α2-3- or α2-6- linked sialic acids. Many of these lactosaminoglycans are fucosylated. These structures are present on both neutrophil glycoproteins and glycolipids. To examine the possible role of these glycans in GMP-140 recognition, cells were treated with endo-β-galactosidase, which hydrolyzes the β1-4 linkage between galactose and N-acetylglucosamine (Galβ1-AGlcNAc) in unbranched polylactosaminyl side chains of glycoproteins. Pretreatment of fixed neutrophils with up to 0.2 U/ml of *E . freundii* endo-β-galactosidase for 30 min, or up to 0.4 U/ml of *B. fragilis* endo-β-galactosidase for 60 min, had no effect on specific binding of GMP-140. The lack of effect of these enzymes on binding is consistent with the GMP-140 recognition structure not being on a polylactosmine side chain. Alternatively and perhaps more likely, the relevant side chains may not be susceptible to enzymatic hydrolysis under these conditions. Highly fucosylated and/or branched polylactosaminoglycan may be resistant to hydrolysis by this enzyme, although branched polylactosaminoglycans are not present in neutrophils. In addition, a single lactosaminoglycan linked directly to the core of an N- or O-linked structure would be resistant to the enzyme.

### Example 6: Requirements of sialyated fucosylated structure for binding of GMP-140.

The following studies were done by Richard Cummings at the University of Georgia using Chinese Hamster Ovary cells, or CHO cells. The cell lines that were used included Ade-C cells and Ade-C cells permanently transfected with specific glycosyl transferases. The Ade-C cells were selected because they contain very low levels of endogenous α(1,3)fucosyltransferases; these cells are denoted as wild type cells in the studies to be described. The transfected cells express certain types of oligosaccharides not present on the wild type cells. Another CHO cell line used, designated Lec8 CHO, is deficient in the transporter for UDPGal and the cells consequently lack galactosylated and sialylated glycoconjugates (Deutscher and Hirschberg, J. Biol. Chem. 261:96-100, 1986). The oligosaccharide structures present on some of these cells and the cell types are shown in Table III. The relevant cells are: wild type (CHO), Neo Lewis (Neo Lew), Neo Lewis related (Neo Lew (rel)), and Lec8 CHO (Lec8).

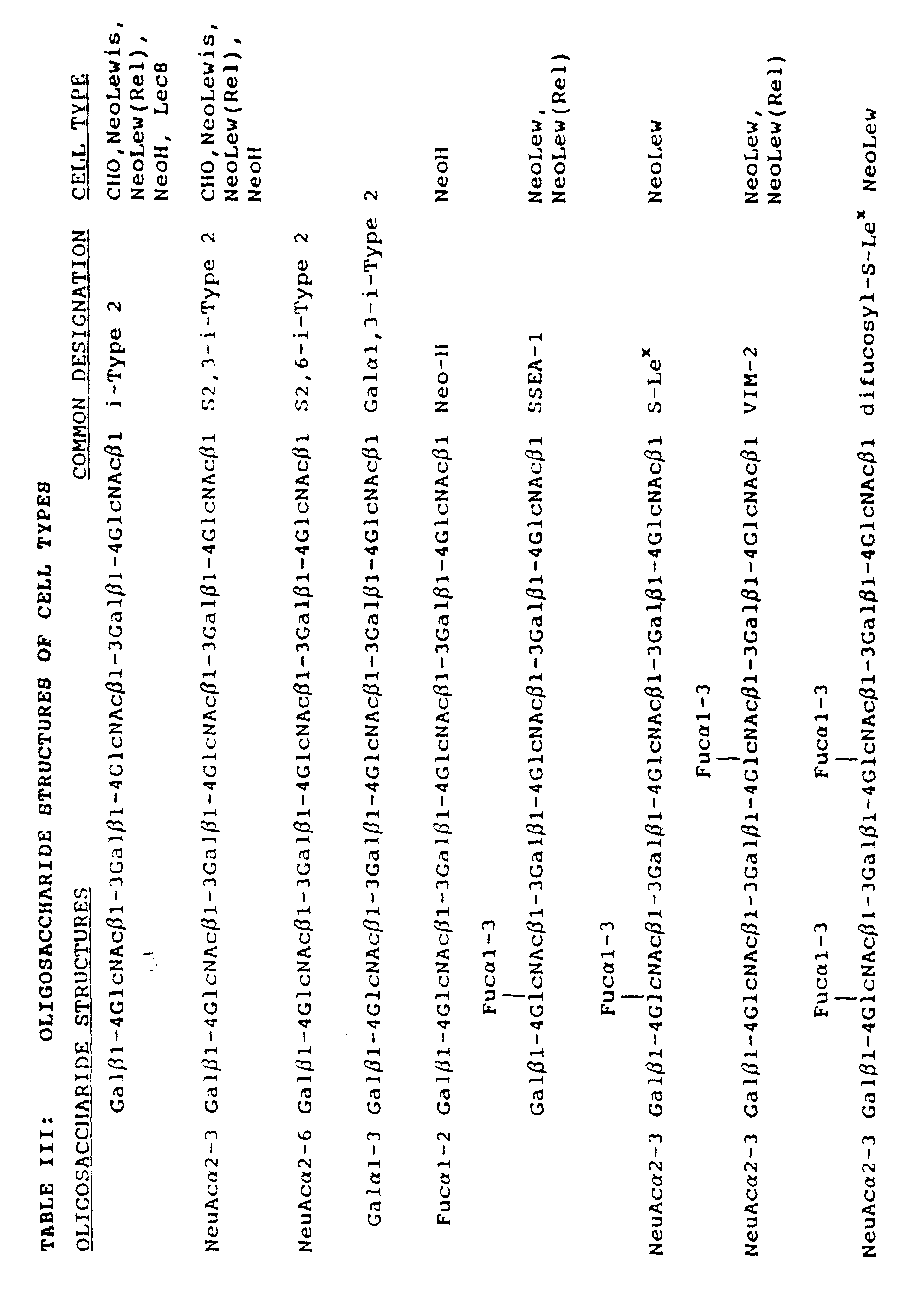

The following methods were used to culture and transfect the cells:

The CHO line Ade-C (Oates and Patterson, Som. Cell Genet. 3:561-577 (1977); Van Keuren et al., Am. J. Hum. Genet. 38:793-804 (1986)) was grown in α-modified Eagle's medium supplemented with 10% fetal calf serum. Transfected CHO cells were grown in media supplemented with G418 (GIBCO) at 400 µg/ml (active drug).

The Neo Lewis CHO cells were prepared by stably transfecting Ade-C CHO cells with DNA encoding an α(1,3/1,4)fucosyltransferase as previously described (Lowe et al., Cell 63:475-484, 1990; the cells were referred to as CHO-FT in this reference). The Neo Lewis (rel) cells were prepared by stably transfecting wild type CHO cells with similar methods with DNA encoding an α(1,3)fucosyltransferase that catalyzes transfer of GDP fucose only to nonsialylated lactosaminoglycans; this transferase is described in Lowe et al., Cell 63:475-484, 1990. Both of the transfected cell lines were gifts from Dr. John Lowe, University of Michigan.

### Results and Conclusions relating to the carbohydrate bound by the selectins including GMP-140.

The structures produced by wild type CHO and transfected CHO cells are shown in Table III. The wild type CHO cells express repeating Galβ1,4GlcNAcβ1,3 disaccharide units, some of which have a terminal sialic acid (NeuAc) linked α2,3. They do not have α2,6-linked sialic acid linkages and they do not have fucose linkages. In addition, they synthesize Type II structures (3Galβ1,4GlcNAcβ1) but not Type I structures (3Galβ1,3GlcNAcβ1). The NeoLewis cells have been transfected with a cDNA encoding an α1,3(4) fucosyltransferase, which uses GDP fucose as a donor and catalyzes addition of fucose to Galβ1,4GlcNAc-R to yield Galβ1,4(Fucα1,3)GlcNAc-R; this is the Le^{x} structure (sometimes also known as the SSEA-1 antigen). The same enzyme transfers fucose to the sialylated substrate NeuAcα2,3Galβ,3 1,4GlcNAc-R to yield NeuAcα2,3Galβ1,4(Fucα1,3)GlcNAc-R; this is the sialyl Le^{x} structure. Two other related structures, VIM-2 and difucosyl Le^{x}, are also made by the NeoLewis cells, as shown in the table, as well as a variety of other sialylated polyfucosylated poly-N-acetyllactosamine-type structures. The NeoLewis related cells have been transfected with a different fucosyl transferase, an α1,3 fucosyltransferase which catalyzes transfer of GDP fucose only to nonsialylated substrates of Galβ1,4GlcNAc-R. This yields the Le^{x} structure (see table III).

To test the ability of these cells to interact with GMP-140, a slight modification of the adhesion assay used for neutrophils and HL60 cells (J.-G. Geng et al, Nature 343:757-760, 1990) was used with purified GMP-140 and monoclonal antibodies to GMP-140. GMP-140 was immobilized on plastic wells in increasing concentrations and the wells were then blocked with albumin-containing buffer. CHO or HL-60 cells, metabolically labeled with [³⁵S]methionine, were added to the wells in the presence or absence of Ca²⁺, and adhesion was measured by solubilizing the bound cells and quantitating the radioactivity.

The results of the binding assays are shown in Figures 4A (CHO binding to GMP-140); 4B (Lec 8 CHO binding to GMP-140); 4C (NeoLewis CHO binding to GMP-140); and 4D (HL60 cell binding to GMP-140).

The HL-60 cells bound specifically to GMP-140-coated wells in a Ca²⁺-dependent manner, as described by Geng, et al, Nature 343:757-760 (1990). The wild type CHO cells, the Lec 8 CHO cells and the NeoLewis rotated CHO cells did not bind. However, like the HL-60 cells, the NeoLewis CHO cells bound avidly to immobilized GMP-140 in a Ca²⁺-dependent manner. The adhesion was specific, because it was prevented by G1, a blocking monoclonal antibody to GMP-140, but not by S12, a nonblocking antibody, as shown in Figure 5. The adhesion was critically dependent on sialic acid, because treatment of the NeoLewis CHO cells with neuraminidase from *Vibrio cholera* abolished binding. Pretreatment of the NeoLewis CHO cells with trypsin reduced binding by 60%, suggesting that at least a substantial fraction of the oligosaccharide ligands for GMP-140 on the cells are carried by a protein(s).

These data confirm that the oligosaccharide ligand for GMP-140 is a sialylated fucosylated structure. The sialic acid linkage must be α2,3 to Gal, because the CHO cells do not have α2,6 linkages. The fucose linkage must be α1,3 to a GlcNAc to which a Gal has been attached by a β1,4 linkage. Possible structures include sialyl Le^{x} itself, difucosyl sialyl Le^{x}, a longer polyfucosylated polylactosaminoglycan variant of sialyl Le^{x}, or a branched structure containing elements of the above components. Le^{x} itself does not provide the necessary affinity or specificity for binding. VIM-2, a sialylated structure lacking a Fuc linked to the GlcNAc closest to the terminal sialic acid, may have affinity for GMP-140, since this structure is present on the Neolewis related cells which do not bind to GMP-140. However, the quantities of VIM-2 on the Neo Lewis related cells are not known; if they are low, then the cells might not bind well even though there is some affinity of VIM-2 for GMP-140.

Although another study has indicated that high concentrations of Le^{x} inhibit adhesion, LNFIII, which includes the Le^{x} trisaccharide, in concentrations up to 300 µM has absolutely no effect on binding of neutrophils to purified, immobilized GMP-140.

### Example 7: Demonstration of differences in binding of ligands by GMP-140 and ELAM-1.

Despite data indicating that the ligands for ELAM-1 and GMP-140 are the same or very similar, there are two lines of evidence that this is not the case.

First, adhesion of neutrophils to COS cells transfected with cDNAs encoding either GMP-140 or ELAM-1 shows specific adhesion to both types of transfected cells. As noted by Geng, et al, Nature (1990), adhesion to GMP-140-transfected cells was blocked by G1, a monoclonal antibody to GMP-140, and adhesion to ELAM-1-transfected cells was blocked by H18/7, a monoclonal antibody to ELAM-1. However, while fluid-phase GMP-140 blocked adhesion to GMP-140-transfected cells, it had no effect on adhesion to ELAM-1-transfected cells. (Figure 6)

Second, a human carcinoma cell line, HT-29, which contains abundant amounts of sialyl Le^{x}, binds to ELAM-1-transfected cells, but not to GMP-140-transfected cells. (Figure 7)

The data in Figures 6 and 7 suggest that GMP-140 and ELAM-1 recognize ligands of somewhat different structure and/or that they differ in the affinity with which they recognize identical ligands. It is possible that GMP-140 and ELAM-1 each binds to a range of related oligosaccharide structures with different degrees of affinities.

The methods and materials used for the studies comparing binding by ELAM-1 and GMP-140 were as follows:

### Cell isolation and culture

Human neutrophils were isolated from normal volunteers using Monopoly resolving media (Flow Labs) as described by Moore, et al., J. Cell Biol. 112, 491-499 (1991). Human HL-60 promyelocytic cells and HT-29 human colon carcinoma cells were obtained from the American Type Culture Collection (Rockville, MD). HL-60 cells were maintained in RPMI-1640/10% fetal bovine serum. HT-29 cells were maintained in culture in McCoy's 5a medium supplemented with 10% fetal calf serum (fcs). COS-7 cells were maintained in Dulbecco's Modified Eagles Media (HG-DMEM) supplemented with 10% calf serum.

### COS7 cell transfection and neutrophil rosetting assay.

Full-length cDNAs encoding GMP-140 or ELAM-1 were inserted into the CDM8 as described by Geng, et al., Nature (1990). COS7 cells were grown to approximately 80% confluency in 10 cm petri dishes in high glucose DMEM (Gibco) supplemented with 10% calf serum (HG-DMEM/10% CS). Fifty µl of Transfectin™ reagent (BRL Life Technologies, Inc.) was combined with 20 µg cDNA in 50 µl water or water alone and allowed to stand for 15 min at room temperature. After the COS cells were washed twice with 3 ml Opti-MEM™ I Reduced Serum Serum Media (BRL Life Technogies, Inc.), the cDNA-lipofectin reagent mixture was added and incubated overnight at 37°C in a 5% CO₂ atmosphere. Six ml HG-DMEM/10% CS was added and the cells incubated for an additional 24 hours. The monolayers were then washed once with HBSS without Ca⁺² and Mg⁺², the cells detached with 0.02% EDTA, pelleted by centrifugation, then resuspended in 12 ml HG-DMEM/10% CS. Two ml of the cell suspension were plated into each well of a 6-well tissue culture (Corning) containing 3 ml HG-DMEM/10% CS and grown for an additional 24 hours. Prior to adhesion assays the wells were washed twice with HBSS. Duplicate wells were incubated with 0.5 ml HBSS containing 30 µg/ml of G1 F(ab')₂, or H18/7 F(ab')₂ or buffer alone for 30 min at 22°C. One ml freshly isolated human neutrophils (2 x 10⁶/ml in HBSS) that was incubated for 30 min in the presence of 30 µg/ml GMP-140 or diluent, were then added to the monolayers and incubated for 20 min at 22°C.

One ml of freshly isolated human neutrophils or ³⁵S-methionine-labeled HT-29 cells (2 x 10⁶ in HBSS/1% HSA) was added and incubated for 20 min at 22°C. In some experiments neutrophils were incubated with purified GMP-140 (10 µg/ml final concentration) for 30 min at 22°C prior to the adhesion assay.

To assay cell adhesion, after five washes with 5 ml HBSS/1% HSA, adherent neutrophils were solubilized with 200 µl 0.5% hexadecyltrimethyl ammonium bromide in 50 mM potassium phosphate pH 6.0. The number of adherent neutrophils was assayed in duplicate using a myeperoxidase assay as described by Geng, et al., Nature 343, 757-760 (1990). To assay HT-29 cell adhesion, adherent cells were solubilized with 1% Triton X100 and quantitated by liquid scintillation counting. Prior to addition of dtergent, the monolayers were examined by phase contrast microscopy to confirm that they were adequately washed and that the COS cell monolayer remained intact.

### Results

GMP-140 inhibits neutrophil adhesion to COS7 cells transfected with cDNA encoding GMP-140 but not to cells transfected with cDNA encoding ELAM-1. COS7 cells were either mock transfected or transfected with cDNAs encoding GMP-140 or ELAM-1. The ability of purified GMP-140, G1 F(ab')₂ or H18/7 F(ab')₂ (all at 10 µg/ml, final concentration) to inhibit neutrophil rosetting to transfected COS7 cells is shown in Figure 6. The data represent the results from two independent transfection experiments. For each transfection, adhesion assays were performed on duplicate monolayers in the presence or absence of GMP-140 and either G1 F(ab')₂ or H18/7 F(ab')₂. Results are expressed as the number of neutrophils bound (mean ± SD).

The results clearly demonstrate that neutrophils bind to COS cells transfected with cDNAs encoding either ELAM-1 or GMP-140 and that the binding is inhibited by appropriate monoclonal antibodies: the anti-ELAM-1 antibody (H18/7) blocks binding ofneutrophils to ELAM-1-transfected cells and the anti-GMP-140 antibody (G1) blocks binding of neutrophils to GMP-140-transfected COS cells. However, fluid-phase GMP-140, while completely blocking neutrophil adhesion to GMP-140-transfected COS cells, has no effect on neutrophil adhesion to ELAM-1-transfected COS cells.

The transfected COS cells were then used to assess differences in binding of HT-29 cells, which contain large amounts of the sialyl Le^{x} structure. The results, shown in Figure 7, demonstrate that HT-29 cells bind avidly to ELAM-1-tranfected cells but not at all to GMP-140-transfected cells. Therefore, interactions of HT-29 cells with GMP-140- and ELAM-1-tranfected COS cells are not identical, even though GMP-140 and ELAM-1 both recognize oligosaccharide structures containing α(2,3)sialylated, α(1,3)fucosylated lactosaminoglycans.

### Example 8: Characterization of the protein component of the GMP-140 ligand on neutrophils.

Treatment of neutrophils with trypsin abolished specific GMP-140 binding, indicating that the predominant ligand for GMP-140 on neutrophils is surface glycoprotein rather than glycospingolipid. Trypsin treatment of HL-60 cells and NeoLewis CHO cells also significantly reduced their adhesion to GMP-140, as shown in Figure 8, indicating that glycoprotein components are also major ligands for GMP-140 on these cells. A glycolipid ligand would not be expected on the NeoLewis CHO cells, since the simple glycolipids synthesized by CHO cells would not be substrates for the transfected fucosyltransferase. The surface proteins bearing the oligosacharide st .ctures recognized by GMP-140 are unlikely to be the same in human myeloid cells and chinese hamster ovary cells. This suggests that high affinity binding of GMP-140 to its ligand does not require a protein-protein interaction.

For trypsin treatment, NeoLewis CHO cells suspended in HEPES buffer A were incubated for 10 min at 37°C with 0.1% DPCC-trypsin. Control cells were incubated under identical conditions with DPCC-trypsin that had been irreversibly inactivated with DFP. After trypsin treatment, cells were chilled on ice and DFP added to 2 mM final concentration to inactivate the enzyme. After treatment with trypsin the cells were washed twice with ice-cold HEPES buffer A prior to assay.

In the case of neutrophils, it has been established that a major glycoprotein recognized by GMP-140 has an apparent Mr of approximately 120,000 as analyzed by SDS-PAGE under reducing conditions. A plasma membrane fraction of human neutrophils was prepared and the material analyzed by "ligand blotting." The material was fractionated by SDS-PAGE, transferred to Immobilon membranes, and probed with [¹²⁵]GMP-140. Consistent binding of labeled GMP-140 to a 120-kD band under reducing conditions was observed. The binding is specific, because it is Ca²⁺-dependent, blocked by antibody G1 but not S12, and eliminated by prior treatment of the membrane with neuraminidase. This protein is bound quantitatively on a wheat germ agglutinin affinity column, indicating that it contains extensively sialylated oligosaccharides.

The protein binds to and can be eluted from an affinity column of GMP-140 coupled to Affigel™. The partially purified protein stains poorly with silver and Coomassie blue. The protein may represent a heavily O-glycosylated protein known as leukosialin, which has a similar apparent Mr and staining pattern on SDS polyacrylamide gels. In addition, treatment of the protein with low doses of neuraminidase, which does not remove all the sialic acid from the protein, results in slower mobility on gels, a pattern consistent with partial desiaylation of certain heavily O-glycosylated proteins.

There may be other proteins on myeloid cells which carry the oligosaccharide ligand for GMP-140. As determined by ligand blotting, the 120-kDa glycoprotein may represent the most abundant ligand and/or the structure that binds with greatest affinity to GMP-140.

### Preparation of Diagnostic and Therapeutic Agents from Peptides Derived from the Lectin Domain of GMP-140 or Carbohydrates Interacting with GMP-140.

The glycoprotein of the invention has a variety of applications as diagnostic reagents and, potentially, in the treatment of numerous inflammatory disorders.

### Diagnostic Reagents.

The GMP-140 binding glycoprotein can also be used for the detection of human disorders in which GMP-140 ligands might be defective. Such disorders would most likely be seen in patients with increased susceptibility to infections in which leukocytes might not be able to bind to activated platelets or endothelium. Cells to be tested, usually leukocytes, are collected by standard medically approved techniques and screened. Detection systems include ELISA procedures, binding of radiolabeled antibody to immobilized activated cells, flow cytometry, or other methods known to those skilled in the arts.

Inhibition of binding in the presence and absence of the lectin domain peptides can be used to detect defects or alterations in selectin binding. Such disorders would most likely be seen in patients with increased susceptibility to infections in which leukocytes would have defective binding to platelets and endothelium because of deficient leukocyte ligands for GMP-140.

A similar approach can be used to determine qualitative or quantitative disorders of GMP-140. The glycoprotein is labeled and tested for its ability to bind to GMP-140 on activated platelets from patients with disorders in which GMP-140 might be defective.

### Clinical Applications.

Since GMP-140 has several functions related to leukocyte adherence, inflammation, and coagulation, clinically, compounds which interfere with binding of GMP-140 and/or the other selectins, including ELAM-1 and LEU-8, such as the GMP-140 peptides or carbohydrates, can be used to modulate these responses.

For example, the glycoprotein can be used to competitively inhibit leukocyte adherence by competitively binding to GMP-140 receptors on the surface of activated platelets or endothelial cells. This kind of therapy would be particularly useful in acute situations where effective, but transient, inhibition of leukocyte-mediated inflammation is desirable. Chronic therapy by infusion of the glycoprotein may also be feasible in some circumstances.

An inflammatory response may cause damage to the host if unchecked, because leukocytes release many toxic molecules that can damage normal tissues. These molecules include proteolytic enzymes and free radicals. Examples of pathological situations in which leukocytes can cause tissue damage include injury from ischemia and reperfusion, bacterial sepsis and disseminated intravascular coagulation, adult respiratory distress syndrome, tumor metastasis, rheumatoid arthritis and atherosclerosis.

Reperfusion injury is a major problem in clinical cardiology. Therapeutic agents that reduce leukocyte adherence in ischemic myocardium can significantly enhance the therapeutic efficacy of thrombolytic agents. Thrombolytic therapy with agents such as tissue plasminogen activator or streptokinase can relieve coronary artery obstruction in many patients with severe myocardial ischemia prior to irreversible myocardial cell death. However, many such patients still suffer myocardial neurosis despite restoration of blood flow. This "reperfusion injury" is known to be associated with adherence of leukocytes to vascular endothelium in the ischemic zone, presumably in part because of activation of platelets and endothelium by thrombin and cytokines that makes them adhesive for leukocytes (Romson et al., Circulation 67: 1016-1023, 1983). These adherent leukocytes can migrate through the endothelium and destroy ischemic myocardium just as it is being rescued by restoration of blood flow.

There are a number of other common clinical disorders in which ischemia and reperfusion results in organ injury mediated by adherence of leukocytes to vascular surfaces, including strokes; mesenteric and peripheral vascular disease; organ transplantation; and circulatory shock (in this case many organs might be damaged following restoration of blood flow).

Bacterial sepsis and disseminated intravascular coagulation often exist concurrently in critically ill patients. They are associated with generation of thrombin, cytokines, and other inflammatory mediators, activation of platelets and endothelium, and adherence of leukocytes and aggregation of platelets throughout the vascular system. Leukocyte-dependent organ damage is an important feature of these conditions.

Adult respiratory distress syndrome is a devastating pulmonary disorder occurring in patients with sepsis or following trauma, which is associated with widespread adherence and aggregation of leukocytes in the pulmonary circulation. This leads to extravasation of large amounts of plasma into the lungs and destruction of lung tissue, both mediated in large part by leukocyte products.

Two related pulmonary disorders that are often fatal are in immunosuppressed patients undergoing allogeneic bone marrow transplantation and in cancer patients suffering from complications that arise from generalized vascular leakage resulting from treatment with interleukin-2 treated LAK cells (lymphokine-activated lymphocytes). LAK cells are known to adhere to vascular walls and release products that are presumably toxic to endothelium. Although the mechanism by which LAK cells adhere to endothelium is not known, such cells could potentially release molecules that activate endothelium and then bind to endothelium by mechanisms similar to those operative in neutrophils.

Tumor cells from many malignancies (including carcinomas, lymphomas, and sarcomas) can metastasize to distant sites through the vasculature. The mechanisms for adhesion of tumor cells to endothelium and their subsequent migration are not well understood, but may be similar to those of leukocytes in at least some cases. The association of platelets with metastasizing tumor cells has been well described, suggesting a role for platelets in the spread of some cancers.

Platelet-leukocyte interactions are believed to be important in atherosclerosis. Platelets might have a role in recruitment of monocytes into atherosclerotic plaques; the accumulation of monocytes is known to be one of the earliest detectable events during atherogenesis. Rupture of a fully developed plaque may not only lead to platelet deposition and activation and the promotion of thrombus formation, but also the early recruitment of neutrophils to an area of ischemia.

Another area of potential application is in the treatment of rheumatoid arthritis.

In these clinical applications, the glycoprotein in an appropriate pharmaceutical carrier, is preferably administered intravenously where immediate relief is required. The glycoprotein can also be administered intramuscularly, intraperitoneally, subcutaneously, orally, as the glycoprotein, conjugated to a carrier molecule, or in a drug delivery device. The glycoprotein can additionally be modified chemically to increase their *in vivo* half-life.

The glycoprotein can be isolated from cells expressing the glycoprotein, either naturally or as a result of genetic engineering as described in the transfected Cos cell examples, or, preferably, by synthetic means. These methods are known to those skilled in the art. In addition, a large number of glycosyltransferases have been cloned (J.C. Paulson and K.J. Colley, J. Biol. Chem. 264:17615-17618, 1989). Accordingly, workers skilled in the art can use a combination of synthetic chemistry and enzymatic synthesis to make pharmaceuticals or diagnostic reagents.

Glycoproteins of the invention that are biologically active are those which inhibit binding of neutrophils and monocytes to GMP-140, or which inhibit leukocyte adhesion to endothelium that is mediated by ELAM-1 and/or the homing receptor.

Suitable pharmaceutical vehicles for administration to a patient are known to those skilled in the art. For parenteral administration, the glycoprotein will usually be dissolved or suspended in sterile water or saline. For enteral administration, the peptide or carbohydrate will be incorporated into an inert carrier in tablet, liquid, or capsular form. Suitable carriers may be starches or sugars and include lubricants, flavorings, binders, and other materials of the same nature. The GMP-140 peptide or carbohydrate can also be administered locally at a wound or inflammatory site by topical application of a solution or cream.

Alternatively, the glycoprotein may be administered in liposomes or microspheres (or microparticles). Methods for preparing liposomes and microspheres for administration to a patient are known to those skilled in the art. U.S. Patent No. 4,789,734 describe methods for encapsulating biological materials in liposomes. Essentially, the material is dissolved in an aqueous solution, the appropriate phospholipids and lipids added, along with surfactants if required, and the material dialyzed or sonicated, as necessary. A good review of known methods is by G. Gregoriadis, Chapter 14. "Liposomes", Drug Carriers in Biology and Medicine pp. 287-341 (Academic Press, 1979), the teachings of which are incorporated herein. Microspheres formed of polymers or proteins are well known to those skilled in the art, and can be tailored for passage through the gastrointestinal tract directly into the bloodstream. Alternatively, the glycoprotein carbohydrate can be incorporated and the microspheres, or composite of microspheres, implanted for slow release over a period of time, ranging from days to months. See, for example, U.S. Patent No. 4,906,474, 4,925,673, and 3,625,214.

The subject glycoproteins are generally active when administered parenterally in amounts above about 1 µg/kg of body weight. For treatment of most inflammatory disorders, the dosage range will be between 0.1 to 30 mg/kg of body weight. A dosage of 70 mg/kg may be required for some of the glycoproteins characterized in the examples. This dosage will be dependent, in part, on whether one or more glycoproteins are administered. As discussed with respect to binding of the three regions of the lectin domain, a synergistic effect may be seen with combinations of glycoproteins from different, or overlapping, regions of the lectin domain, or in combination with glycoproteins derived from the EGF domain of GMP-140.

The glycoproteins should be active when administered parenterally or by other means. The amounts needed will be based on concentrations required for inhibition of GMP-140 binding to myeloid cells in in *vitro* assays and on the clearance rates of the infused carbohydrates. This dosage will be dependent, in part, on whether one or more carbohydrates are administered. A synergistic effect may be seen with combinations of carbohydrates, or with multivalent forms of the natural ligand, or derivatives thereof, designed to increase affinity and/or avidity for GMP-140.

The glycoproteins can also be coated onto substrates for use as prosthetics that are implanted into the body to prevent leukocyte adhesion to platelets or endothelium.

Modifications and variations of the present invention, methods for modulating binding reactions involving the GMP-140 ligand, will be obvious to those skilled in the art from the foregoing detailed description. Such modifications and variations are defined by the technical features of the appended claims.

## Claims

1. A glycoprotein comprising a carbohydrate portion comprising a sialylated, fucosylated lactosaminoglycan, the carbohydrate portion O-linked to the glycoprotein, and having affinity binding specific to GMP-140 wherein binding of the glycoprotein ligand to GMP-140 is blocked by the G1 monoclonal antibody to GMP-140 and not blocked by the S12 monoclonal antibody to GMP-140.

2. The glycoprotein of claim 1 wherein the affinity binding specific to GMP-140 is further defined as Ca²⁺-dependent.

3. The glycoprotein of claim 1 wherein the carbohydrate portion comprises a sialylated Lewis^{x} group.

4. The glycoprotein of claim 3 wherein the sialylated Lewis^{x} group further comprises NeuAcα2,3Galβ1,4(Fucα1,3) GlcNAcβ1-R wherein R is a protein, a glycoprotein, or a carbohydrate structure.

5. The glycoprotein of any one of claims 1, 2, 3 and 4 wherein the carbohydrate portion comprises an α2,3-sialylated lactosaminoglycan.

6. The glycoprotein of any one of claims 1 to 5 wherein the carbohydrate portion comprises an α1,3-fucosylated, α2,3-sialylated lactosaminoglycan.

7. The glycoprotein of any one of claims 1 to 6 where the carbohydrate further comprises a difucosyl sialyl Le^{x} group or a polyfucosylated polylactosaminoglycan group.

8. A glycoprotein obtainable by the expression of an α(1,3) fucosyltransferase in a cell line stably transfected with a cDNA encoding the α(1,3) fucosyltransferase, the glycoprotein comprising a carbohydrate protein comprising a sialylated fucosylated lactosaminoglycan, the carbohydrate portion O-linked to the glycoprotein and having affinity binding specific to GMP-140, wherein binding of the glycoprotein ligand to GMP-140 is blocked by the G1 monoclonal antibody to GMP-140 and not blocked by the S12 monoclonal antibody to GMP-140.

9. The glycoprotein of claim 8 wherein the carbohydrate portion further comprises an α1,3-fucosylated, α2,3-sialylated lactosaminoglycan.

10. The glycoprotein of either claims 8 or 9 wherein the carbohydrate portion further comprises a difucosyl sialyl Lewis^{x} group or a polyfucosylated sialyl Lewis^{x} polylactosaminoglycan group.

11. The glycoprotein of any one of claims 1 to 10 which inhibits the binding of GMP-140 to neutrophils.

12. The glycoprotein of any one of claims 1 to 11 having a molecular weight of about 120,000 Daltons as asertained by SDS-PAGE under reducing conditions.

13. A method of producing the glycoprotein of claim 8, comprising the steps of: stably transfecting a cell line with a cDNA encoding an α(1,3) fucosyltransferase; and causing the cell line to express α(1,3) fucosyltransferase, wherein the glycoprotein is produced, the glycoprotein comprising a carbohydrate portion comprising a sialylated, fucosylated lactosaminoglycan, the carbohydrate portion O-linked to the glycoprotein and having affinity binding specific to GMP-140, wherein binding of the glycoprotein ligand to GMP-140 is blocked by the G1 monoclonal antibody to GMP-140 and not blocked by the S12 monoclonal antibody to GMP-140.

14. The method of claim 13 wherein in the step of causing the cell line to express α(1,3) fucosyltransferase, the carbohydrate portion of the glycoprotein produced further comprises a difucosyl sialyl Lewis^{x} group or a polyfucosylated sialyl Lewis^{x} polylactosaminoglycan.

15. An *in vitro* process comprising:
causing GMP-140 to bind to a GMP-140 binding protein wherein said GMP-140 binding protein is expressed in a host cell transfected with a cDNA encoding an α(1,3) fucosyltransferase and wherein the expressed GMP-140 binding protein comprises a carbohydrate portion comprising a sialylated, fucosylated lactosaminoglycan, the carbohydrate portion O-linked to the glycoprotein and has affinity binding specificity to GMP-140, wherein binding of the glycoprotein ligand to GMP-140 is blocked by the G1 monoclonal antibody to GMP-140 and not blocked by the S12 monoclonal antibody to GMP-140.

16. Use of a cell line stably transfected with a cDNA encoding the α(1,3) fucosyltransferase in the manufacture of a glycoprotein wherein the glycoprotein comprising a carbohydrate protein comprising a sialylated fucosylated lactosaminoglycan, the carbohydrate portion O-linked to the glycoprotein and having affinity binding specific to GMP-140, wherein binding of the glycoprotein ligand to GMP-140 is blocked by the G1 monoclonal antibody to GMP-140 and not blocked by the S12 monoclonal antibody to GMP-140.

17. Use of the glycoprotein of any one of claims 1 to 12 in the manufacture of a medicament for the treatment of an inflammatory process or coagulation process.

## Patentansprüche

1. Glycoprotein, umfassend einen Kohlenhydratanteil, umfassend eine sialyliertes, fucosyliertes Lactosaminoglycan, wobei der Kohlenhydratanteil über Sauerstoff mit dem Glycoprotein verknüpft ist und eine für GMP-140 spezifische Bindungsaffinität aufweist, wobei das Binden des Glycoproteinliganden an GMP-140 durch den G1-monoklonalen Antikörper an GMP-140 blockiert wird und nicht durch den S12-monoklonalen Antikörper an GMP-140 blokkiert wird.

2. Glycoprotein nach Anspruch 1, wobei die für GMP-140 spezifische Bindungsaffinität weiter als Ca²⁺-abhängig definiert ist.

3. Glycoprotein nach Anspruch 1, wobei der Kohlenhydratanteil eine sialylierte Lewis^{x}-Gruppe umfaßt.

4. Glycoprotein nach Anspruch 3, wobei die sialylierte Lewis^{x}-Gruppe weiter NeuAcα2,3Galβ1,4(Fucα1,3) GlcNAcβ1-R umfaßt, wobei R ein Protein, ein Glycoprotein oder eine Kohlenhydratstruktur ist.

5. Glycoprotein nach einem der Ansprüche 1, 2, 3 und 4, wobei der Kohlenhydratanteil ein α2,3-sialyliertes Lactosaminoglycan umfaßt.

6. Glycoprotein nach einem der Ansprüche 1 bis 5, wobei der Kohlenhydratanteil ein α1,3-fucosyliertes, α2,3-sialyliertes Lactosaminoglycan umfaßt.

7. Glycoprotein nach einem der Ansprüche 1 bis 6, wobei das Kohlenhydrat weiter eine Difucosyl-Sialyl-Lex-Gruppe oder eine polyfucosylierte Polylactosaminoglycan-Gruppe umfaßt.

8. Glycoprotein, erhältlich durch die Expression einer α(1,3)-Fucosyltransferase in einer Zelllinie, welche stabil mit einer α(1,3)-Fucosyltransferase kodierenden cDNA transfiziert ist, wobei das Glykoprotein ein Kohlenhydratprotein umfaßt, welches ein sialyliertes, fucosyliertes Lactosaminglycan umfaßt, wobei der Kohlenhydratanteil über Sauerstoff mit dem Glycoprotein verknüpft ist und eine für GMP-140 spezifische Bindungsaffinität aufweist, wobei das Binden des Glycoproteinliganden an GMP-140 durch den G1-monoklonalen Antikörper an GMP-140 blockiert wird und nicht durch den S12-monoklonalen Antikörper an GMP-140 blockiert wird.

9. Glycoprotein nach Anspruch 8, wobei der Kohlenhydratanteil weiter ein α 1,3- fucosyliertes, α2,3-sialyliertes Lactosaminoglycan umfaßt.

10. Glycoprotein nach Anspruch 8 oder 9, wobei der Kohlenhydratanteil weiter eine Difucosyl-Sialyl-Lewis^{x}-Gruppe oder eine polyfucosylierte Sialyl-Lewis^{x}-Polylactosaminoglycan-Gruppe umfaßt.

11. Glycoprotein nach einem der Ansprüche, welches die Bindung von GMP-140 an Neutrophile hemmt.

12. Glycoprotein nach einem der Ansprüche 1 bis 11 mit einem Molekulargewicht von etwa 120 000 Dalton, ermittelt durch SDS-PAGE unter reduzierenden Bedingungen.

13. Verfahren zum Herstellen des Glycoproteins nach Anspruch 8, umfassend die Schritte des stabilen Transfizierens einer Zelllinie mit einer α (1,3)Fucosyltransferase kodierenden cDNA und des Bewirkens der Zelllinie α(1,3)Fucosyltransferase zu exprimieren, wodurch das Glycoprotein hergestellt wird, wobei das Glycoprotein einen Kohlenhydratanteil umfaßt, welcher ein sialyliertes, fucosyliertes Lactosaminglycan umfaßt, wobei der Kohlenhydratanteil über Sauerstoff mit dem Glycoprotein verknüpft ist und eine für GMP-140 spezifische Bindungsaffinität aufweist, wobei das Binden des Glycoproteinliganden an GMP-140 durch den G1-monoklonalen Antikörper an GMP-140 blockiert wird und nicht durch den S12-monoklonalen Antikörper an GMP-140 blockiert wird.

14. Verfahren nach Anspruch 13, worin in dem Schritt des Bewirkens der Zelllinie, α(1,3)-Fucosyltransferase zu exprimieren, der Kohlenhydratanteil des hergestellten Glycoproteins weiter eine Difucosyl-Sialyl-Lewis^{x}-Gruppe oder ein polyfucosyliertes Sialyl-Lewis^{x}-Polylactosaminoglycan umfaßt.

15. *In vitro*-Verfahren, umfassend das Bewirken, GMP-140 an ein GMP-140 bindendes Protein zu binden, wobei das GMP-140 bindende Protein in einer Wirtszelle exprimiert wird, welche mit einer α(1,3)Fucosyltransferase kodierenden cDNA transfiziert ist, und wobei das exprimierte GMP-140 bindende Protein einen Kohlenhydratanteil umfaßt, welcher ein sialyliertes, fucosyliertes Lactosaminoglycan umfaßt, wobei der Kohlenhydratanteil über Sauerstoff mit dem Glycoprotein verknüpft ist und die für GMP-140 spezifische Bindungsaffinität aufweist, wobei das Binden des Glycoproteinliganden an GMP-140 durch den G1-monoklonalen Antikörper an GMP-140 blockiert wird und nicht durch den S12-monoklonalen Antikörper an GMP 140 blockiert wird.

16. Verwendung einer Zelllinie, welche stabil mit einer die α(1,3)Fucosyltransferase codierenden cDNA transfiziert ist, zur Herstellung eines Glycoproteins, wobei das Glycoprotein ein Kohlenhydratprotein umfaßt, welches ein sialyliertes, fucosyliertes Lactosaminoglycan umfaßt, wobei der Kohlenhydratanteil über Sauerstoff mit dem Glycoprotein verknüpft ist und eine für GMP-140 spezifische Bindungsaffinität aufweist, wobei das Binden des Glycoproteinliganden an GMP-140 durch den G1-monoklonalen Antikörper an GMP-140 blockiert wird und nicht durch den S1-monoklonalen Antikörper an GMP-140 blockiert wird.

17. Verwendung des Glycoproteins nach einem der Ansprüche 1 bis 12 zur Herstellung eines Medikaments für die Behandlung eines Entzündungsprozesses oder eines Gerinnungsprozesses.

## Revendications

1. Glycoprotéine comprenant une portion glucidique comprenant un lactosaminoglycane fucosylé sialylé, la portion glucidique étant reliée par O à la glycoprotéine, et ayant une liaison d'affinité spécifique avec GMP-140 où la liaison du ligand glycoprotéine à GMP-140 est bloquée par l'anticorps monoclonal G1 dirigé contre GMP-140 et n'est pas bloquée par l'anticorps monoclonal S12 dirigé contre GMP-140.

2. Glycoprotéine de la revendication 1 dans laquelle la liaison d'affinité spécifique avec GMP-140 est définie en outre comme étant Ca²⁺-dépendante.

3. Glycoprotéine de la revendication 1 dans laquelle la portion glucidique comprend un groupe Lewis^{x} sialylé.

4. Glycoprotéine de la revendication 3 dans laquelle le groupe Lewis^{x} sialylé comprend en outre NeuAcα2,3Galβ1,4(Fucα1,3) GlcNAcβ1-R où R est une protéine, une glycoprotéine, ou une structure glucidique.

5. Glycoprotéine de l'une quelconque des revendications 1, 2, 3 et 4 dans laquelle la portion glucidique comprend un lactosaminoglycane α2,3-sialylé.

6. Glycoprotéine de l'une quelconque des revendications 1 à 5 dans laquelle la portion glucidique comprend un lactosaminoglycane α2,3-sialylé α1,3-fucosylé.

7. Glycoprotéine de l'une quelconque des revendications 1 à 6 dans laquelle le glucide comprend en outre un groupe difucosyl-sialyl-Le^{x} ou un groupe polylactosaminoglycane polyfucosylé.

8. Glycoprotéine pouvant être obtenue par l'expression d'une α(1,3)fucosyltransférase dans une lignée cellulaire transfectée de manière stable avec un ADNc codant pour 1'α(1,3)fucosyltransférase, la glycoprotéine comprenant une protéine glucidique comprenant un lactosaminoglycane fucosylé sialylé, la portion glucidique étant reliée par O à la glycoprotéine, et ayant une liaison d'affinité spécifique avec GMP-140, où la liaison du ligand glycoprotéine à GMP-140 est bloquée par l'anticorps monoclonal G1 dirigé contre GMP-140 et n'est pas bloquée par l'anticorps monoclonal S12 dirigé contre GMP-140.

9. Glycoprotéine de la revendication 8 dans laquelle la portion glucidique comprend en outre un lactosaminoglycane α2,3-sialylé α1,3-fucosylé.

10. Glycoprotéine de la revendication 8 ou 9 dans laquelle la portion glucidique comprend en outre un groupe difucosyl-sialyl-Lewis^{x} ou un groupe polylactosaminoglycane sialyl-Lewis^{x} polyfucosylé.

11. Glycoprotéine de l'une quelconque des revendications 1 à 10 qui inhibe la liaison de GMP-140 aux neutrophiles.

12. Glycoprotéine de l'une quelconque des revendications 1 à 11 ayant une masse moléculaire d'environ 120 000 daltons telle qu'établie par SDS-PAGE dans des conditions réductrices.

13. Procédé de production de la glycoprotéine de la revendication 8, comprenant les étapes consistant à : transfecter de manière stable une lignée cellulaire avec un ADNc codant pour une α(1,3)fucosyltransférase; et amener la lignée cellulaire à exprimer l'α(1,3)fucosyltransférase, moyennant quoi la glycoprotéine est produite, la glycoprotéine comprenant une portion glucidique comprenant un lactosaminoglycane fucosylé sialylé, la portion glucidique étant reliée par O à la glycoprotéine, et ayant une liaison d'affinité spécifique avec GMP-140, où la liaison du ligand glycoprotéine à GMP-140 est bloquée par l'anticorps monoclonal G1 dirigé contre GMP-140 et n'est pas bloquée par l'anticorps monoclonal S12 dirigé contre GMP-140.

14. Procédé de la revendication 13 dans lequel dans l'étape consistant à amener la lignée cellulaire à exprimer l'α(1,3)fucosyltransférase, la portion glucidique de la glycoprotéine produite comprend en outre un groupe difucosyl-sialyl-Lewis^{x} ou un polylactosaminoglycane sialyl-Lewis^{x} polyfucosylé.

15. Procédé *in vitro* consistant à :
amener GMP-140 à se lier à une protéine liant GMP-140, où ladite protéine liant GMP-140 est exprimée dans une cellule hôte transfectée avec un ADNc codant pour une α(1,3)fucosyltransférase, et où la protéine liant GMP-140 exprimée comprend une portion glucidique comprenant un lactosaminoglycane fucosylé sialylé, la portion glucidique étant reliée par O à la glycoprotéine et a une spécificité de liaison d'affinité pour GMP-140, où la liaison du ligand glycoprotéine à GMP-140 est bloquée par l'anticorps monoclonal G1 dirigé contre GMP-140 et n'est pas bloquée par l'anticorps monoclonal- S12 dirigé contre GMP-140.

16. Utilisation d'une lignée cellulaire transfectée de manière stable avec un ADNc codant pour l'α(1,3)fucosyltransférase dans la fabrication d'une glycoprotéine où la glycoprotéine comprend une protéine glucidique comprenant un lactosaminoglycane fucosylé sialylé, la portion glucidique étant reliée par O à la glycoprotéine, et a une liaison d'affinité spécifique avec GMP-140, où la liaison du ligand glycoprotéine à GMP-140 est bloquée par l'anticorps monoclonal G1 dirigé contre GMP-140 et n'est pas bloquée par l'anticorps monoclonal S12 dirigé contre GMP-140.

17. Utilisation de la glycoprotéine de l'une quelconque des revendications 1 à 12, dans la fabrication d'un médicament destiné au traitement d'un processus inflammatoire ou d'un processus de coagulation.
